(19) Europäisches Patentamt
European Patent Office
Office européen des brevets

(11) **EP 4 523 626 B1**

(12) **EUROPEAN PATENT SPECIFICATION**

(45) Date of publication and mention
of the grant of the patent:
**27.05.2026 Bulletin 2026/22**

(21) Application number: **23908696.0**

(22) Date of filing: **14.07.2023**

(51) International Patent Classification (IPC):
*A61B 5/304* (2021.01)    *A61B 5/00* (2006.01)
*A61B 5/0245* (2006.01)    *A61B 5/30* (2021.01)

(52) Cooperative Patent Classification (CPC):
**A61B 5/30; A61B 5/0006; A61B 5/0245;**
**A61B 5/7207; A61B 5/721; A61B 5/7214;**
**A61B 5/7225**

(86) International application number:
**PCT/CN2023/107582**

(87) International publication number:
**WO 2025/015474 (23.01.2025 Gazette 2025/04)**

(54) **SIGNAL PROCESSING SYSTEM AND METHOD**

SIGNALVERARBEITUNGSSYSTEME UND -VERFAHREN

SYSTÈME ET PROCÉDÉ DE TRAITEMENT DE SIGNAUX

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**19.03.2025 Bulletin 2025/12**

(73) Proprietor: **Shenzhen Shokz Co., Ltd.**
**Shenzhen, Guangdong 518108 (CN)**

(72) Inventors:
• **ZHOU, Xin**
**Shenzhen, Guangdong 518108 (CN)**
• **ZHANG, Yuxiang**
**Shenzhen, Guangdong 518108 (CN)**
• **NING, Yixuan**
**Shenzhen, Guangdong 518108 (CN)**
• **LIAO, Fengyun**
**Shenzhen, Guangdong 518108 (CN)**
• **QI, Xin**
**Shenzhen, Guangdong 518108 (CN)**

(74) Representative: **Wang, Bo**
**Panovision IP**
**Ebersberger Straße 3**
**85570 Markt Schwaben (DE)**

(56) References cited:
EP-A1- 3 858 232        CN-A- 101 073 493
CN-A- 105 476 628       CN-A- 110 974 210
CN-A- 113 271 854       CN-A- 114 680 905
CN-A- 115 023 178       US-A1- 2007 142 735
US-A1- 2012 157 867     US-A1- 2023 216 486

• AN XIANG ET AL: "Adaptive Motion Artifact
Reduction in Wearable ECG Measurements
Using Impedance Pneumography Signal",
SENSORS, vol. 22, no. 15, 23 July 2022
(2022-07-23), CH, pages 5493, XP093211512,
ISSN: 1424-8220, DOI: 10.3390/s22155493
• TEXAS INSTRUMENTS: "ADS129x Low-Power, 2-
Channel, 24-Bit Analog Front-End for
Biopotential Measurements", 24 April 2020
(2020-04-24), pages 1 - 82, XP093211528,
Retrieved from the Internet <URL:https://www.ti.
com/lit/ds/symlink/ads1292r.pdf?
ts=1727906509402&ref_url=https%3A%2F%
2Fwww.ti.com%2Fproduct%2FADS1292R>
[retrieved on 20241003]

EP 4 523 626 B1

- ZHANG HUANQIAN ET AL: "A multi-channel electrode tissue impedance detection approach for motion artifact suppression in ambulatory electrocardiography", COMPUTING IN CARDIOLOGY, N/A, 6 September 2015 (2015-09-06), pages 117 - 120, XP032865119, ISSN: 2325-8861, ISBN: 978-1-4799-0884-4, [retrieved on 20160216], DOI: 10.1109/CIC.2015.7408600
- KHALILI MATIN ET AL: "Motion artifacts in capacitive ECG monitoring systems: a review of existing models and reduction techniques", 20 July 2024 (2024-07-20), DE, XP093211261, ISSN: 0140-0118, Retrieved from the Internet <URL:https://link.springer.com/article/10.1007/s11517-024-03165-1/fulltext.html> [retrieved on 20241003], DOI: 10.1007/s11517-024-03165-1
- LYU YINGJUN: "The Research and Design of Multichannel Collection System for Feeble Physiology Signals", MICROCOMPUTER INFORMATION, WEIJISUANJI XINXI, CHINA, vol. 21, no. 7-1, 15 September 2005 (2005-09-15), China
, pages 110 - 112, XP093263186, ISSN: 1008-0570

## Description

### TECHNICAL FIELD

The present disclosure relates to the field of signal processing, and in particular, to signal processing systems and methods.

### BACKGROUND

When collecting physiological signals (e.g., ECG signals) of an object, the motion (e.g., running, jumping, etc.) of the object may cause the collected physiological signals to contain motion artifacts (MA), and thus be in error from real physiological signals. To eliminate the motion artifacts, in addition to electrodes for collecting the physiological signals, sensors are usually used to obtain motion artifacts. Then the motion artifacts contained in the physiological signals can be eliminated based on the collected motion artifacts. However, the physiological signals obtained based on this method are still not sufficiently accurate because the sensors work on a different principle than the electrodes and the motion artifacts identified by the sensors are different from the motion artifacts collected by the electrodes.

Therefore, it is desired to propose a signal processing system and method that can more accurately eliminate the interference of motion artifacts on physiological signals.

Relevant prior art is disclosed in AN XIANG ET AL: "Adaptive Motion Artifact Reduction in Wearable ECG Measurements Using Impedance Pneumography Signal", which relates to use adaptive filtering, a specially designed ECG device, and an Impedance Pneumography (IP) data acquisition system to combat motion artifacts. Relevant prior art is disclosed in Texas Instruments: "ADS129x Low-Power, 2-Channel, 24-Bit Analog Front-End for Biopotential Measurements", which relates to the datasheet of the ADS 1292R chip. Relevant prior art is disclosed in ZHANG HUANQIAN ET AL: "A multi-channel electrode tissue impedance detection approach for motion artifact suppression in ambulatory electrocardiography", which relates to evaluate an approach for measuring the electrode tissue impedance (ETI) variation for motion artifacts suppression application. Relevant prior art is disclosed in EP 3858232A1, which relates to a sensing system and method using a sense electrode arrangement for coupling to a surface of a body such that the sense electrode arrangement and the body (and the spacing between them) define a coupling capacitance.

### SUMMARY

The invention is set out in the appended set of claims.

## BRIEF DESCRIPTION OF THE DRAWINGS

The present disclosure is further illustrated in terms of exemplary embodiments. These exemplary embodiments are described in detail with reference to the drawings. These embodiments are not limiting, and in these embodiments, the same numbering denotes the same structure, where:

FIG. 1 is a schematic diagram illustrating an exemplary application scenario of a signal processing system according to some embodiments of the present disclosure;

FIG. 2 is a block diagram illustrating an exemplary signal collection device according to the present invention;

FIG. 3 is a schematic diagram illustrating an exemplary transmission path of a physiological signal when the physiological signal is collected according to some embodiments of the present disclosure;

FIG. 4 is a schematic diagram illustrating electrocardiogram signals collected at different input impedances according to some embodiments of the present disclosure;

FIG. 5 is a schematic diagram illustrating an exemplary transmission path of a motion artifact signal when the motion artifact signal is collected according to some embodiments of the present disclosure;

FIG. 6 is a schematic diagram illustrating an exemplary processing circuit according to some embodiments of the present disclosure;

FIG. 7 is a schematic diagram illustrating electrocardiogram signals including motion artifacts caused by a relative displacement of a horizontal action according to some embodiments of the present disclosure;

FIG. 8 is a schematic diagram illustrating electrocardiogram signals including motion artifacts caused by a relative displacement of a vertical action according to some embodiments of the present disclosure;

FIG. 9 is a schematic diagram illustrating electrocardiogram signals including motion artifacts caused by compression according to some embodiments of the present disclosure;

FIG. 10A is a schematic diagram illustrating a processing circuit of a signal processing system according to some embodiments of the present disclosure;

FIG. 10B is a schematic diagram illustrating a processing circuit of a signal processing system according to some embodiments of the present disclosure;

FIG. 10C is a schematic diagram illustrating a processing circuit of a signal processing system according to some embodiments of the present disclosure;

FIG. 11 is a schematic diagram illustrating another processing circuit of a signal processing system according to some embodiments of the present disclosure;

FIG. 12 is a schematic diagram illustrating a processing circuit of another signal processing system according to some embodiments of the present disclosure;

FIG. 13 is a flowchart illustrating a signal processing process according to the present invention; and

FIG. 14 is a flowchart illustrating another signal processing process according to the present invention.

## DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and a person of ordinary skill in the art can apply the present disclosure to other similar scenarios based on the accompanying drawings without creative labor. Unless obtained from the context or the context illustrates otherwise, the same numeral in the drawings refers to the same structure or operation.

It should be understood that the terms "system," "device," "unit," and/or "module" are used herein as a way to distinguish between different components, elements, parts, sections, or assemblies at different levels. However, the words may be replaced by other expressions if other words accomplish the same purpose.

As shown in the present disclosure and the claims, unless the context clearly suggests an exception, the words "a," "an," "one," "one kind," and/or "the" do not refer specifically to the singular, but may also include the plural. Generally, the terms "including," and "comprising" suggest only the inclusion of clearly identified steps and elements that do not constitute an exclusive list, and the method or device may also include other steps or elements.

Flowcharts are used in the present disclosure to illustrate operations performed by a system in accordance with embodiments of the present disclosure. It should be appreciated that the preceding or following operations are not necessarily performed in an exact sequence. Instead, steps can be processed in reverse order or simultaneously. Also, it is possible to add other operations to these processes or remove a step or steps from them.

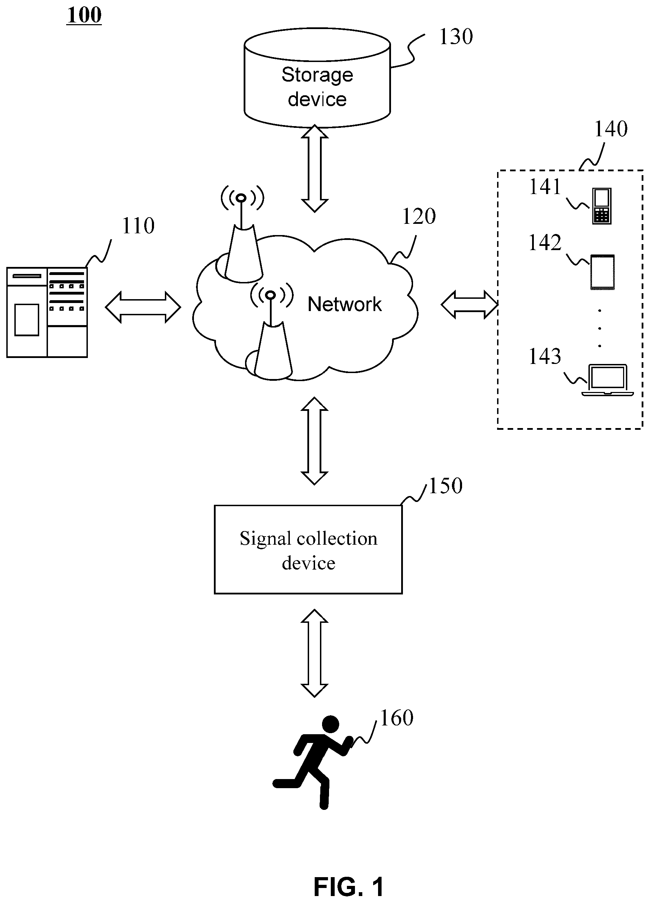

FIG. 1 is a schematic diagram illustrating an exemplary application scenario of a signal processing system according to some embodiments of the present disclosure.

According to FIG. 1, in some embodiments, a signal processing system (hereinafter referred to as the system 100) may include a processing device 110, a network 120, a storage device 130, a terminal device 140, a signal collection device 150, and an object 160. Components in the system 100 may be connected in a variety of manners. For example, the signal collection device 150 may be connected to the storage device 130 and/or the processing device 110 via the network 120, or may be directly connected to the storage device 130 and/or the processing device 110. As another example, the storage device 130 may be directly connected to the processing device 110 or connected via the network 120. As another example, the terminal device 140 may be connected to the storage device 130 and/or the processing device 110 via the network 120 or may be directly connected to the storage device 130 and/or the processing device 110.

In some embodiments, the system 100 may collect and process physiological signals by implementing methods and/or processes disclosed in the present disclosure.

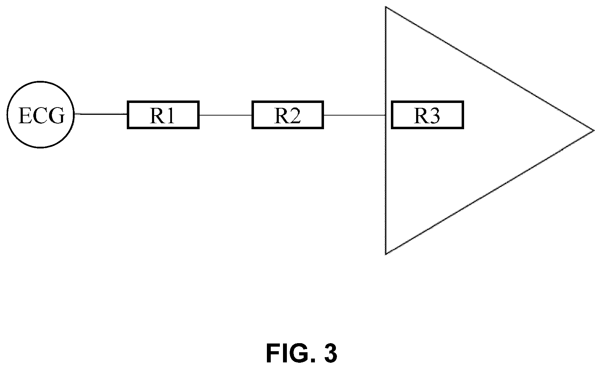

The physiological signal refers to a bioelectrical signal originating from the human body, for example, an electrocardiogram (ECG) signal, an electromyographic (EMG) signal, or the like, and collected by a signal collection device (e.g., the signal collection device 150). The physiological signal may be a mixed signal with a motion artifact (MA) signal and a pure physiological signal. The pure physiological signal refers to a real physiological signal of the object 160 obtained after filtering out the motion artifact signal based on the processing of the physiological signal. More descriptions regarding the process for collecting and processing the physiological signal can be found in FIG. 3, FIG. 5, FIG. 10 A to FIG. 14 and relevant descriptions thereof.

The processing device 110 may process data and/or information obtained from the signal collection device 150, the storage device 130, the terminal device 140, and/or other components of the system 100. In some embodiments, the processing device 110 may obtain the physiological signals of the object 160 from one or more of the signal collection device 150, the storage device 130, or the terminal device 140, and process the physiological signals. In some embodiments, the processing device 110 may obtain pre-stored computer instructions from the storage device 130 and execute the computer instructions to implement the signal processing method described herein.

In some embodiments, the processing device 110 may be a single server or a group of servers. The group of servers may be centralized or distributed. In some embodiments, the processing device 110 may be local or remote. For example, the processing device 110 may access information and/or data from the signal collection device 150, the storage device 130, and/or the terminal device 140 via the network 120. As another example, the processing device 110 may be directly connected to the signal collection device 150, the storage device 130, and/or the terminal device 140 to access information and/or data. Merely by way of example, the processing device 110 may be directly connected to the signal collection device 150 through a wired (e.g., a wire, a circuit board, etc.) connection, and the processing device 110 may directly access data or a signal collected by the signal collection device 150 for processing. In some embodiments, the processing device 110 may be implemented on a cloud platform. For example, the cloud platform may include a private cloud, a public cloud, a hybrid cloud, a community cloud, a distributed cloud, an inter-cloud cloud, a multi-cloud, etc., or any combination thereof. In some embodiments, the processing device 110 may be implemented on a terminal device (e.g., the terminal device 140).

The network 120 may connect various components of the system 100 and/or connect the system 100 to an external resource. The network 120 may enable communication between various components of the system 100, or with other parts outside the system 100, thereby facilitating the exchange of data and/or information. For example, the processing device 110 may obtain the physiological signals from the signal collection device 150 and/or the storage device 130 via the network 120. As another example, the processing device 110 may obtain a user operation instruction from the terminal device 140 via the network 120. Exemplary operation instructions may include, but are not limited to, setting information about an object (e.g., gender, age, height, weight, disease history, etc.), etc.

In some embodiments, the network 120 may be any form of wired or wireless network, or any combination thereof. Merely by way of example, the network 120 may include a cable network, a wired network, a fiber optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, etc., or any combination thereof. In some embodiments, the network 120 may include at least one network access point, and at least one component of the system 100 may be connected to the network 120 via the access point to exchange data and/or information. For example, data, such as a collected physiological signal, may be transmitted via the network 120.

The storage device 130 may store data, instructions, and/or any other information. In some embodiments, the storage device 130 may store data obtained from the signal collection device 150, the processing device 110, and/or the terminal device 140. For example, the storage device 130 may store the physiological signals collected by the signal collection device 150. In some embodiments, the storage device 130 may store data and/or instructions for the processing device 110 to execute or use to accomplish exemplary methods described in the present disclosure. In some embodiments, the storage device 130 may include a mass memory, a removable memory, a volatile read-write memory, a read-only memory (ROM), or the like, or any combination thereof. Exemplary mass memory may include disks, optical disks, solid-state disks, or the like. In some embodiments, the storage device 130 may be implemented on a cloud platform. Merely by way of example, the cloud platform may include a private cloud, a public cloud, a hybrid cloud, a community cloud, a distributed cloud, an on-premises cloud, a multi-tiered cloud, etc., or any combination thereof.

In some embodiments, the storage device 130 may be connected to the network 120 to communicate with at least one other component of the system 100 (e.g., the signal collection device 150, the processing device 110, the terminal device 140). At least one component of the system 100 may access data, instructions, or other information stored in the storage device 130 via the network 120. In some embodiments, the storage device 130 may be directly connected or in communication with one or more components of the system 100 (e.g., the signal collection device 150, the terminal device 140). In some embodiments, the storage device 130 may be part of the signal collection device 150 and/or the processing device 110.

The terminal device 140 may receive, send and/or display data. Received data may include data collected by the signal collection device 150, data stored by the storage device 130, data generated by the processing device 110, or the like. For example, data received and/or displayed by the terminal device 140 may include the physiological signals collected by the signal collection device 150, a pure physiological signal obtained by the processing device 110 after processing the physiological signals, or the like. Sent data may include user input data and instructions, etc. For example, the terminal device 140 may send a user-input operation instruction to the signal collection device 150 via the network 120 to control the signal collection device 150 to perform corresponding data collection.

In some embodiments, the terminal device 140 may include a mobile device 141, a tablet computer 142, a laptop computer 143, etc., or any combination thereof. For example, the mobile device 141 may include a cell phone, a personal digital assistant (PDA), a medical mobile terminal, a wearable device (e.g., a smartwatch, a bracelet, etc.), etc., or any combination thereof. In some embodiments, the terminal device 140 may include an input device (e.g., a keyboard, a touch screen), an output device (e.g., a display, a speaker), or the like. In some embodiments, the processing device 110 may be part of the terminal device 140.

The signal collection device 150 may be a device that collects the physiological signals of the object 160 in different time periods. In some embodiments, the signal collection device 150 may include at least one electrode, and the at least one electrode may be used to fit at least one body part of the object 160 to collect the physiological signals of the object 160. In some embodiments, the signal collection device 150 may have a separate power supply, which may send collected data to other components (e.g., the processing device 110, the storage device 130, the terminal device 140) via a wired or wireless (e.g., Bluetooth, WiFi, etc.) manner. In some embodiments, one or more components of the system 100 may be implemented on the signal collection device 150. For example, the processing device 110, the storage device 130, or the like may be included in the signal collection device 150.

The object 160 may be an object using the signal collection device 150, for example, a patient, an experimenter, an athlete, a fitness enthusiast, an elderly person, or the like whose physiological signals need to be collected.

It should be noted that the foregoing description of the system 100 is intended to be exemplary and illustrative only and does not limit the scope of the present disclosure. For a person skilled in the art, various corrections and changes can be made to the system 100 under the guidance of the present disclosure. However, these corrections and changes remain within the scope of the present disclosure.

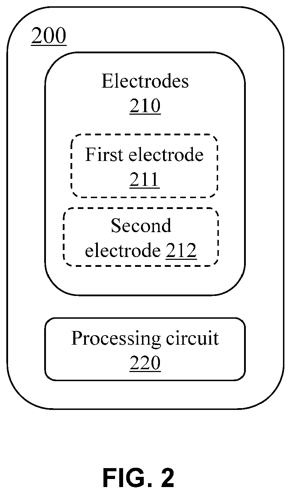

FIG. 2 is a block diagram illustrating an exemplary signal processing system according to the present invention. In some embodiments, the signal collection device 150 described in FIG.1 may be implemented by a signal processing system 200. As illustrated in FIG. 2, the signal processing system 200 includes a group of electrodes 210 and a processing circuit 220.

The group of electrodes 210 is configured to fit the human skin and collect physiological signals. As shown in FIG. 2, the group of electrodes 210 includes at least a first electrode 211 and a second electrode 212. The group of electrodes 210 may fit different parts of the human body, a part to which the first electrode 211 fits has a first potential and a part to which the second electrode 212 fits has a second potential, and a physiological signal may be determined based on a difference between the first potential and the second potential.

The physiological signals may include bioelectrical signals such as electrocardiogram signals, electromyographic signals, etc., and a detailed description regarding the physiological signals can be found in FIG. 1.

In some embodiments, the physiological signals may be electrocardiogram (ECG) signals. Based on this, in some embodiments, the first electrode 211 and the second electrode 212 may fit two positions at different distances from the heart of the object 160, and potentials of the two positions (the first potential and the second potential) are collected, respectively. An ECG signal may be determined based on a difference between the potentials of the two positions.

In some embodiments, the first electrode 211 and the second electrode 212 may be disposed on both sides of the median sagittal plane of the human body and detect potentials (the first potential and the second potential) on both sides of the median sagittal plane of the human body, respectively. The ECG signal may be determined based on a difference between the potentials on both sides of the median sagittal plane of the human body.

The median sagittal plane of the human body is a plane that passes through the midline of the human body and divides the body into two equal or nearly equal parts. The midline of the human body may be determined by a line from the tip of the nose of the human body to the middle of the two nipples, or from the middle of the two nipples to the middle of the umbilicus of the abdomen, or from the middle of the umbilicus of the abdomen to the middle of the symphysis pubis.

In some embodiments, the first electrode 211 and the second electrode 212 may fit the skin of the lumbar region on both sides of the median sagittal plane, respectively. The lumbar region of the human body may be a region of the human body between the lower end of the ribs and the lower end of the ilium, which primarily includes the abdominal and iliac portions. The median sagittal plane of the human body may divide the lumbar region of the human body into a left lumbar region and a right lumbar region, and in some embodiments, the first electrode 211 and the second electrode 212 may fit the skin of the left lumbar region and the right lumbar region, respectively. In some embodiments, the first electrode 211 and the second electrode 212 may fit the skin of the left hip region and the right hip region, respectively. In some embodiments, the first electrode 211 and the second electrode 212 may fit the skin of the left knee and the right knee, respectively. In some embodiments, the first electrode 211 and the second electrode 212 may fit the skin of the left ankle and the right ankle, respectively. It will be appreciated that the first electrode 211 and the second electrode 212 may also fit other parts of the body, such as the skin of the left thigh and the right thigh, the skin of the left calf and the right calf, or the like.

In some embodiments, the physiological signals may be electromyographic signals, and the first electrode 211 and the second electrode 212 may be configured to collect electromyographic signals from the same muscle of the user. The same muscle refers to a single muscle or the same muscle group (e.g., a quadriceps muscle group of the rectus femoris, the lateral femoris, the medial femoris, and the intermediate femoris). For example, when the user wears the signal collection device 150, the first electrode 211 and the second electrode 212 may be proximate to a central region of the muscle in the muscle fiber direction (or referred to as a muscle length direction). The first electrode 211 and the second electrode 212 may be spaced apart along the muscle fiber direction of the muscle and extend in an extension direction perpendicular to the muscle fiber direction, respectively. Different positions in the muscle fiber direction have different potentials, a position of a muscle fiber where the first electrode 211 is located has a first potential, a position of a muscle fiber where a second electrode 212 is located has a second potential, and there is a potential difference between the first potential and the second potential. The electromyographic signals may be determined based on the potential difference.

In some embodiments, the group of electrodes may further include a reference electrode. The reference electrode may be used to provide a reference potential for the first electrode and/or the second electrode, thereby reducing interference of noise.

In some embodiments, the group of electrodes 210 may be secured to a wearable body through a substrate structure. For example, the substrate structure may be made of a flexible insulating material (e.g., resin, soft PVC, silicone, fabric, etc.), and the group of electrodes 210 may be fixedly attached to the substrate structure by pasting, snap-fitting, welding, sewing, hot pressing, etc., and further fixed to the wearable body through the substrate structure. In some embodiments, the substrate structure may be omitted, and the group of electrodes 210 may be integrally molded (e.g., integrally woven, etc.) with the wearable body. When the user wears the signal collection device 150, the group of electrodes 210 may be in contact with the skin of the object.

The processing circuit is configured to read the physiological signals in a time-sharing mode. Reading in the time-sharing mode includes : the processing circuit has different input impedances in different time periods of the time-sharing mode, and the physiological signals read by the processing circuit in the different time periods include motion artifact signals of different proportions. In some embodiments, the different time periods in the time-sharing mode may not overlap. In some embodiments, the time-sharing mode may be achieved by reading (or sampling) the physiological signals of a plurality of channels in a time-sharing multiplexing manner (e.g., using a time-sharing multiplexing switch). The time required for switching the time-sharing multiplexing switch is short (e.g., less than 1 $\mu$s), and the plurality of time periods in the time-sharing mode may be consecutive or near-consecutive time periods. In some embodiments, durations of the plurality of time periods may be the same (i.e., evenly divided) or different. In some embodiments, the duration of one of the plurality of time periods may be a preset value. In some embodiments, different strategies may be used in implementing the time-sharing mode in the time-sharing multiplexing manner, exemplary strategies may include a full reconfiguration signal strategy, a spliced signal strategy, or the like. In different strategies, the duration of a time period of the plurality of time periods may be different. That is, the preset value of the duration may be determined based on a strategy used in the time-sharing multiplexing manner.

For example, taking the full reconfiguration signal strategy as an example, the full reconfiguration signal strategy refers to sampling the signal of each sampling channel by quickly switching the time-sharing multiplexing switch, thereby reconfiguring an original signal at a relatively high sampling frequency. Quickly switching the time-sharing multiplexing switch refers to that a switching frequency of the time-sharing multiplexing switch is greater than a frequency of a target signal to be sampled (e.g., a heart rate signal, an EMG signal, etc.), for example, the sampling frequency (i.e., the switching frequency of the time-sharing multiplexing switch) may be greater than or equal to two times, five times, etc., the frequency of the target signal. Thus, the switching frequency of the time-sharing multiplexing switch may be determined based on the frequency of the target signal, thereby determining the duration of one of the plurality of time periods. For example, the frequency of the target signal may include 10 Hz, and a corresponding switching frequency may be greater than or equal to 20 Hz when the collection is performed through a single channel. If the collection of the signal is performed through n channels, a corresponding switching frequency may be greater than or equal to 20*n Hz. Merely by way of example, the processing circuit 220 may include two signal processing sub-circuits, i.e., the signal collection is performed through two channels, and correspondingly, the switching frequency of the time-sharing multiplexing switch may be greater than or equal to 40 Hz, and the duration of a time period may be less than or equal to 25 ms. Moreover, the switching frequency of the time-sharing multiplexing switch being too quick may result in resource depletion, and the switching frequency is limited by the system capacitance effect, thus the switching frequency may not be too high. For example, the switching frequency is not greater than 100 KHz when a single channel is used for the signal collection, and the duration of a time period may be greater than or equal to 10 $\mu$s. For example, taking a signal collection using the full reconfiguration signal strategy as an example, the switching frequency of the time-division multiplexing switch may be in a range of 40 Hz-200 KHz if a count of channels is n=2, and the duration of a time period may be in a range of 5 $\mu$s-25 ms; or, when the count of the channels is n=2, the switching frequency of the time-division multiplexing switch may be in a range of 80 Hz-100 KHz, and the duration of a time period may be in a range of 10 $\mu$s-12.5 ms; or, when the count of the channels is n=2, the switching frequency of the time-division multiplexing switch may be in a range of 160 Hz-20 KHz, and the duration of a time period may be in a range of 50 $\mu$s-6.25 ms.

As another example, taking the spliced signal strategy as an example, the spliced signal strategy refers to sampling the signal of each sampling channel by slow switching of the time-sharing multiplexing switch. The slow switching of the time-sharing multiplexing switch refers to that the time-sharing multiplexing switch switches at a frequency that is less than the frequency of the target signal to be sampled and greater than an action frequency of the object when the object is in motion. For example, the sampling frequency (i.e., the switching frequency of the time-sharing multiplexing switch) may be greater than or equal to two times, five times, etc., the action frequency of the object, whereby a series of frequency signals may be collected in each time period. Because the switching of the time-sharing multiplexing switch is fast relative to the action frequency, signals collected by a plurality of channels still have reference value. In such cases, the switching frequency of the time-sharing multiplexing switch may be determined based on an action frequency corresponding to the target signal, thereby determining the duration of one of the plurality of time periods. For example, a running action with an action frequency of 3 Hz indicates that the object can complete a running action in 0.33s. Therefore, when analyzing the target signal (e.g., an EMG signal), signals within 0.33 s may be analyzed. Merely by way of example, signals within 0.33 s may be divided into 10 parts for analysis, and the duration of a time period may be 33 ms. In some embodiments, when the target signal is a high-frequency signal and an action is a low-frequency action, e.g., when a fitness action has an action cycle of 2s and a corresponding electromyographic signal is in the order of 100 Hz, the switching frequency of the time-sharing multiplexing switch may be determined based on an action frequency corresponding to the target signal, thereby determining the duration of one of the plurality of time periods. For example, taking the signal collection using the spliced signal strategy as an example, when the count of channels is n=2, the switching frequency of the time-sharing multiplexing switch may be greater than 0.4 Hz, and the duration of a time period may be less than 2.5 s; or, when the count of channels is n=2, the switching frequency of the time-sharing multiplexing switch may be in a range of 0.5 Hz-200 Hz, and the duration of a time period may be in a range of 5 ms-2 s; or, when the count of channels is n=2, the switching frequency of the time-sharing multiplexing switch may be in a range of 1 Hz-100 Hz, and the duration of a time period may be in a range of 10 ms-1 s. In some embodiments, the sampling frequency may be measured based on the processing circuit. For example, the processing circuit may include an analog-digital converter(ADC) circuit, and based on a sampling principle of the ADC, a measurement circuit for measuring the sampling frequency may be brought out at a pin of the ADC circuit. The measurement circuit may be used to measure a change in parameters (e.g., a capacitance, an inductance, a resistor, etc.) when the processing circuit performs the ADC sampling, so as to determine the sampling frequency.

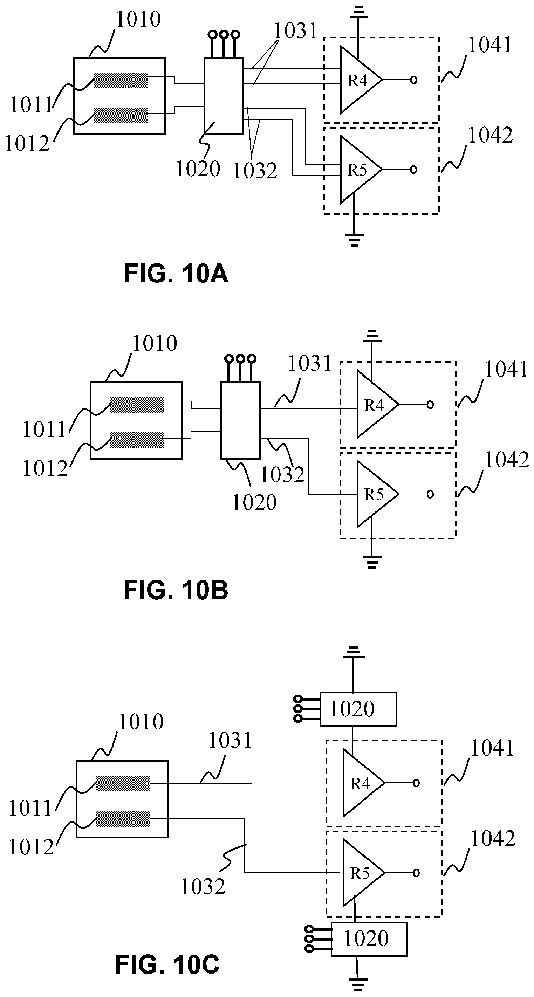

The processing circuit processes the physiological signals based on the motion artifact signals of different proportions. Processing the physiological signals refers to filtering out the motion artifact signals from the physiological signals, thereby obtaining a pure physiological signal without or with fewer motion artifact signals. More descriptions regarding the processing circuit can be found in FIG. 10 A to FIG.12 and relevant descriptions thereof.

During the collection of the physiological signals, the motion (e.g., running, jumping, etc.) of the object causes the motion artifacts in the collected physiological signals. The motion artifacts mainly originate from a skin surface where the electrodes fit, and the motion artifacts may include a half-cell potential when the physiological signals are, for example, ECG signals. The half-cell potential refers to a potential generated due to a change in a position of an electrode and/or a change in the pressure of the electrode on the skin due to the motion, which may cause the motion artifacts in the collected ECG signals.

According to the voltage divider rule of series circuit, the greater the input impedance of the processing circuit, the greater the voltage that the processing circuit divides when collecting the physiological signals.

The voltage divider rule of series circuit may be illustrated by the following equation (1):

$$V_1 = (R_1/(R_1 + R_2 + \cdots + R_n)) \times V. \quad (1)$$

where, $V_1$ denotes a voltage divided by the resistor $R_1$ in the circuit, $(R_1+R_2+\cdots+R_n)$ denotes a total impedance of the circuit including $R_1$, 1, 2, ..., n denote resistor serial numbers, and V denotes a voltage of the power supply (i.e., a total circuit voltage). Therefore, when the impedances of other resistors of the circuit and the total circuit voltage is constant, if the impedance of R1 increases, the voltage $V_1$ divided by $R_1$ will increase.

The physiological signal originates from under the skin, whereas the motion artifact signal originates primarily from the skin surface. A difference between the physiological signal and the motion artifact signal may include that the motion artifact signal is collected without passing through a skin impedance, which is predominantly the stratum corneum. In such cases, the difference between the physiological signal and the motion artifact signal is amplified based on different input impedances of the processing circuit and utilizing the voltage divider rule.

FIG. 3 is a schematic diagram illustrating a transmission path of a physiological signal when the physiological signal is collected according to some embodiments of the present disclosure. As shown in FIG. 3, ECG represents a physiological signal source, R1 is a skin impedance mainly composed of the stratum corneum, R2 is a contact impedance, R3 is an input impedance of a processing circuit (e.g., an impedance between a signal input end of the processing circuit and a reference ground), and a triangle wrapping R3 represents a processing circuit of a signal collection device. The physiological signal source is located inside the body (e.g., the muscle, the heart), the contact impedance R2 refers to an impedance generated when a signal propagates through different media (the electrode and the skin) at the point where the electrode touches the skin. In some embodiments, the contact impedance R2 may also include skin impedance fluctuations, electrode impedance fluctuations, or the like.

Taking the physiological signal being an electrocardiogram (ECG) signal as an example, since the heart is located in the body, the ECG signal to be transmitted to the signal collection device needs to pass through the skin impedance R1 mainly composed of the stratum corneum, then pass through the contact impedance R2, and finally the input impedance of the processing circuit R3. If the input impedance R3 is much larger than the other impedances, a voltage divided by R3 accounts for a larger proportion of a voltage corresponding to the ECG signal (also referred to as an ECG voltage, a magnitude of the ECG voltage characterizes a strength of the ECG signal), and thus the ECG signal may be fully collected by the processing circuit. Conversely, if R3 is much smaller than the other impedances, the ECG voltage may be mainly divided by the other impedances (e.g., R1, R2) in the processing circuit, and then the ECG voltage divided by the input impedance R3 may be very small such that the processing circuit may not fully collect the ECG signal. Therefore, a relatively large (e.g., in the order of 1 GΩ) input impedance is used for physiological signal collection (the collected physiological signal is still a mixed signal containing motion artifacts), and the collected physiological signal includes a relatively sufficient and pure physiological signal.

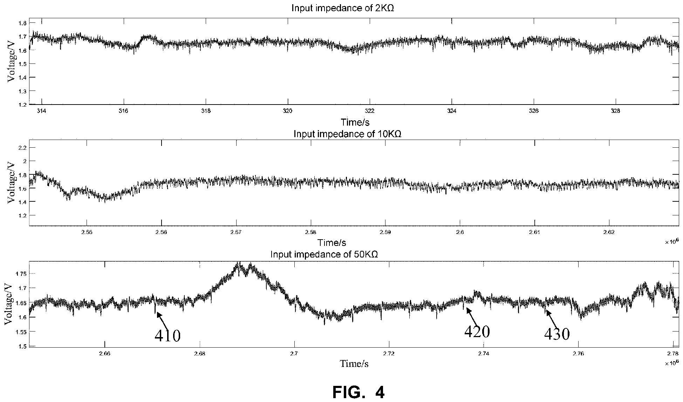

FIG. 4 is a schematic diagram illustrating electrocardiogram (ECG) signals collected at different input impedances according to some embodiments of the present disclosure.

As shown in FIG. 4, ECG signals were measured when the input impedance was 2KΩ, 10KΩ, and 50KΩ, respectively (the regular "burr" in the figure is the ECG signal, and "burr" refers to a peak/valley with an amplitude greater than an average amplitude in an ECG signal map, e.g., a burr 410, a burr 420, and a burr 430 in FIG. 4). From top to bottom in FIG. 4, the ECG becomes gradually apparent as the input impedance increases. Accordingly, increasing the input impedance can increase the proportion of the pure ECG signal in the collected ECG signal, so that the collected ECG signal may mainly include the pure ECG signals.

In some embodiments, the proportion of the pure ECG signal in the collected ECG signal is reduced by reducing the input impedance, so that the collected ECG signal mainly includes the motion artifact signal. In such cases, the pure ECG signal is obtained based on the ECG signal (mainly including the motion artifact signal) collected using the processing circuit with the large input impedance and the ECG signal collected using the processing circuit with the small input impedance. For example, the ECG signal collected using the processing circuit with the large input impedance is subtracted from the ECG signal (mainly including the motion artifact signal) collected using the processing circuit with the small input impedance to obtain the pure ECG signal.

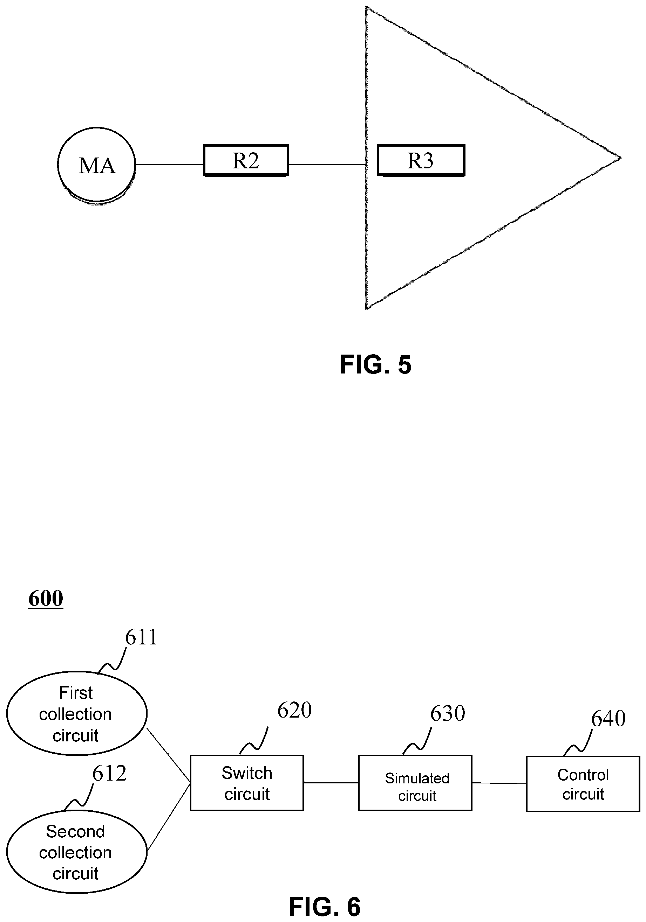

FIG. 5 is a schematic diagram illustrating a transmission path of a motion artifact signal when the motion artifact signal is collected according to some embodiments of the present disclosure. As shown in FIG. 5, MA is a motion artifact source, R2 is the contact impedance, R3 is the input impedance of the processing circuit, and the triangle wrapping R3 represents the processing circuit of the signal collection device. The motion artifact source refers to a source that generates a motion artifact signal (e.g., a change in a potential at a contact point of an electrode caused by a change in a position of the electrode and/or a change in the pressure of the electrode on the skin due to a motion), and since the motion artifact source is located outside the stratum corneum of the skin, the transmission of the motion artifact signal does not need to pass through the skin impedance R1.

On this basis, taking the physiological signal being an ECG signal as an example, by using a relatively small (e.g., in the order of 1KΩ) input impedance R3, a collected ECG signal may include a pure ECG signal of relatively small proportion and a motion artifact signal of a relatively large proportion. The strength of the motion artifact signal (which can be characterized by a voltage corresponding to the motion artifact signal, the voltage corresponding to the motion artifact signal is also referred to as a motion artifact voltage, and the magnitude of the motion artifact voltage characterizes the strength of the motion artifact signal) collected by the input impedance R3 is related to fluctuations of the motion artifact source (e.g., the change in the position of the electrode and/or the change in the pressure of the electrode on the skin due to a motion), a change of the contact impedance R2, etc. When an impedance value of the input impedance R3 decreases, a collected ECG voltage decreases, which in turn causes the motion artifact voltage in the collected ECG voltage to decrease. However, since there is no voltage divider of the skin impedance R1 mainly composed of the stratum corneum, the reduction in the motion artifact voltage due to the decrease of R3 may be less severe than the reduction in the ECG voltage. On the other hand, a change in the magnitude of the contact impedance R2 may cause the magnitude of the motion artifact voltage collected by the input impedance R3 to increase as the input impedance R3 decreases, where the decrease of R3 refers to a decrease within a preset range, for example, a minimum value of R3 within the preset range is in the same order as R2. Specifically, the motion artifact voltage collected by the input impedance R3 may decrease as R3 decreases, but the degree of the decrease of the motion artifact voltage is less than the degree of the decrease of the ECG voltage collected by the input impedance R3. In such cases, the motion artifact signal may be more prominent, so that the change in the magnitude of the contact impedance R2 may cause the motion artifact voltage collected by the input impedance R3 to increase as R3 decreases (it can be understood that the motion artifact signal is more sensitive to a change in R2, and a relatively small change in the magnitude of R2 can result in a relatively large change in the motion artifact signal). Therefore, an input impedance R3 with a small impedance value (e.g., in the order of 1 KΩ) may be used for signal collection, and the collected ECG signal may contain mainly motion artifacts and a small proportion of pure ECG signals.

More specifically, a relationship between the strength of the motion artifact signal collected by the input impedance R3 and the fluctuations of the motion artifact source may be illustrated by equation (2):

$$ma1 = (r_3/(r_2 + r_3)) \times \Delta V. \quad (2)$$

where $ma1$ denotes a motion artifact voltage corresponding to fluctuations of a motion artifact source, $r_2$ denotes an impedance value of the contact impedance R2, $r_3$ denotes an impedance value of the input impedance R3, and $\Delta V$ denotes the fluctuations of the motion artifact source.

The relationship between the strength of the motion artifact signal collected by the input impedance R3 and the change in the magnitude of the contact impedance R2 may be illustrated by equation (3):

$$ma2 = \left(\frac{r_3}{r_2 + \Delta r_2 + r_3}\right) \times V. \quad (3)$$

where $ma2$ denotes a motion artifact voltage corresponding to the change in the impedance of the contact impedance R2, $\Delta r_2$ denotes an amount of change in the impedance value of the contact impedance R2, $r_3$ denotes the impedance value of the input impedance R3, and $V$ denotes a real motion artifact signal.

According to equations (2) and (3) and the above analysis, a relatively small impedance value (e.g., in the order of 1K$\Omega$) of input impedance R3 may be used for signal collection, and the collected ECG signal contains mainly motion artifacts and a small proportion of pure ECG signals. For example, the input impedance R3 may be in the same order as the contact impedance R2. Further, the input impedance R3 may be close to the contact impedance R2. For example, a ratio of the input impedance R3 to the contact impedance R2 may be in a range of 0.1-10, 0.2-5, 0.4-2.5, or the like.

In the present invention, physiological signals and motion artifact signals are separately collected using different input impedances, such that the processing circuit mainly collects ECG signals at a large input impedance, while the ECG signals become small and the motion artifact signals are enhanced at a small input impedance. A correlation algorithm may be used to analyze and calculate the pure physiological signal and denoise the physiological signals.

The processing circuit is used to read physiological signals collected by a group of electrodes in a time-sharing mode. To realize this function, an entire transmission time corresponding to the reading of the physiological signals are divided into a plurality of time periods. The processing circuit has different input impedances in different time periods, so as to read the physiological signals using different input impedances in the different time periods of the time-sharing mode. For example, the transmission time may be divided into n time periods, and the processing circuit has at least two input impedances, with different input impedances in adjacent time periods, e.g., an input impedance in the 1st time period is a, an input impedance in the 2nd time period is b, an input impedance in the 3rd time period is a, and an input impedance in the 4th time period input impedance is b, etc. In some embodiments, the plurality of time periods may be consecutive and non-overlapping. In some embodiments, durations of the plurality of time periods may be the same (i.e., evenly divided) or different. In some embodiments, the duration of a time period may be a preset value. For example, the duration of one of the plurality of time periods may be determined based on a frequency of a target signal. As another example, the duration of one of the plurality of time periods may be determined based on a frequency of an action corresponding to the target signal.

To realize the function that the processing circuit can read the physiological signals in the time-sharing mode, a structure of the processing circuit may be: an electrode module of the processing circuit includes two electrodes (a first electrode and a second electrode), and a circuit connected to the electrode module includes two sub-circuits (referred to as a first sub-circuit and a second sub-circuit), the first sub-circuit has a large input impedance (e.g., an impedance of more than 1 M$\Omega$), the second sub-circuit has a small input impedance (e.g., in the order of 100 K$\Omega$), and both sub-circuits are connected to the electrode module. In such cases, motion artifact signals collected by the second sub-circuit may be highly correlated with motion artifact signals collected by the first sub-circuit. Furthermore, to avoid the impedances of the two sub-circuits from affecting each other, the two sub-circuits may read signals in the time-sharing mode, so as to isolate the input impedances of the two sub-circuits.

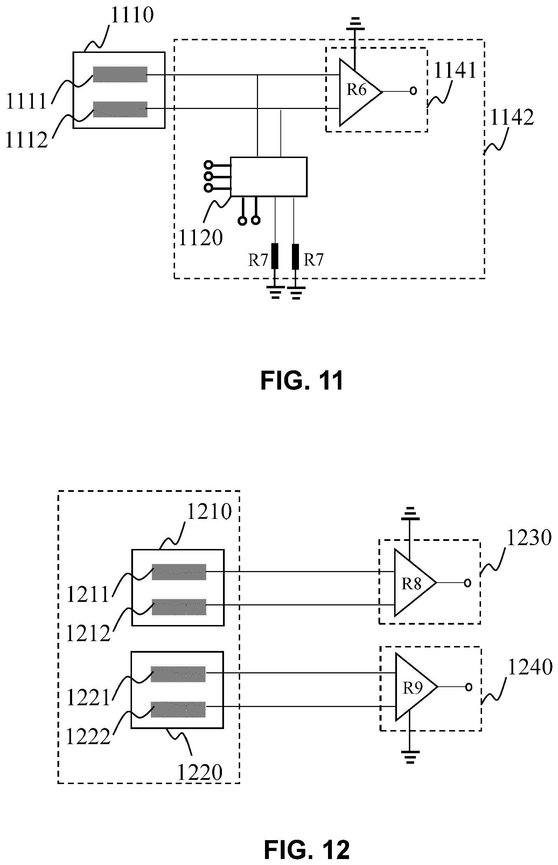

A specific implementation of the processing circuit may include: the processing circuit may include two signal processing sub-circuits (corresponding to the first sub-circuit and the second sub-circuit described above, respectively), the two signal processing sub-circuits having different resistors, i.e., different input impedances. The two sub-circuits may be connected or disconnected from a group of electrodes by a time-sharing multiplexing switch, thereby realizing the reading of signals in the time-sharing mode. For example, in a time period 1, the group of electrodes is connected to the first sub-circuit and disconnected from the second sub-circuit, in a time period 2, the group of electrodes is connected to the second sub-circuit and disconnected from the first sub-circuit, and in a time period 3, the group of electrodes is connected to the first sub-circuit and disconnected from the second sub-circuit, etc. In some embodiments, there is only one circuit, with an additional resistor connected in parallel with an input end of the circuit, and a connection between the additional resistor and the circuit is controlled by a switch, such that the circuit has different input impedances in different time periods. More descriptions can be found in FIG. 10 A, FIG. 10 B, FIG. 11, and relevant descriptions thereof.

In some embodiments of the present disclosure, signals collected by the processing circuit always originate from the same group of electrodes, while the time-sharing multiplexing manner is used to collect signals from the same group of electrodes using different input impedances. In such cases, the MA collected by a circuit with a small input impedance may be highly correlated with the MA collected by a circuit with a normal input impedance, which avoids collection errors caused by connecting different groups of electrodes with different impedances, thereby improving the accuracy of measurement. Physiological signals read in different time periods may include motion artifact signals of different proportions. The processing circuit may process the physiological signals based on the motion artifact signals of different proportions.

In addition to the above implementation manner, two groups of electrodes are used in the processing circuit and connected to the large impedance and the small impedance, respectively for reading signals, and more details can be found in FIG.12 and relevant descriptions thereof.

In the invention, the different input impedances include at least two input impedances, where a ratio of the largest impedance in the at least two input impedances to the smallest input impedance in the two input impedances is not less than 10. For example, a resistor in the circuit may be different in different time periods such that the ratio of the impedances is not less than 10. Merely by way of example, a structure of the circuit may be: input impedances of the two sub-circuits are different, or the input impedance of one sub-circuit is connected in parallel with a resistor and a switch is used to control the connection between the resistor and the sub-circuit so that the ratio of the impedances is not less than 10. In some embodiments, the ratio of the largest input impedance in the two input impedances to the smallest input impedance in the two input impedances at different frequencies may be different. For example, to match a target signal at a relatively low frequency, e.g., at a frequency of 0.5 Hz-100 Hz, the ratio of the largest input impedance in the two input impedances to the smallest input impedance in the two input impedances is not less than 10. As another example, to match a target signal at a relatively high frequency, e.g., when the frequency is greater than 100 Hz, the ratio of the largest input impedance in the two input impedances to the smallest input impedance in the two input impedances may be increased, e.g., not less than $10^3$.

In some embodiments, at a frequency of 50 Hz, the largest input impedance in the two input impedances is not less than 1 MΩ and the smallest input impedance in the two input impedances is not greater than 100 KΩ.

In some embodiments of the present disclosure, by setting the resistors, the ECG signal may be sufficiently collected by the circuit with the large input impedance, the ECG signal collected by the circuit with the small input impedance may be small, and the motion artifacts signal collected by the circuit with the small input impedance may be relatively large, which makes the difference more obvious, thereby improving the processing accuracy of the physiological signals.

In some embodiments, the processing circuit may perform a filtering operation on the motion artifact signals based on a correspondence of physiological signals including the motion artifact signals of different portions, so as to obtain a pure physiological signal, and/or obtain a target physiological signal by performing an enhancement operation on the pure physiological signal. For example, the processing circuit may identify the motion artifact signal based on the physiological signal read at the smallest input impedance, and a signal with a high correlation with the motion artifact signal in the physiological signal read at the largest impedance may be regarded as the motion artifact signal, to be filtered out from the physiological signal read at the largest impedance. Merely by way of example, when motion artifacts are caused by factors such as motion (e.g., running, jumping, etc.), the physiological signal read at the small input impedance may have obvious fluctuations (as shown by range A in FIG. 6), and the processing circuit may identify the signal having the obvious fluctuations and take the signal as the motion artifact signal. Further, the processing circuit may determine a correspondence between the physiological signal read at the large input impedance and the motion artifact signal, perform a filtering operation on the motion artifact signals to obtain a pure physiological signal, and/or obtain a target physiological signal by performing an enhancement operation on the pure physiological signal.

It is to be noted that the above description of the signal processing system is provided only for descriptive convenience, and does not limit the present disclosure to the scope of the cited embodiments. It is to be understood that for a person skilled in the art, after understanding the principle of the processing system, it may be possible to arbitrarily combine the various modules or form a subsystem to be connected to the other modules without departing from this principle. In some embodiments, the electrode 210 and the processing circuit 220 disclosed in FIG. 1 may be different modules or a single module that may realize the functions of two or more modules as described above. For example, the individual modules may share a common storage module, and the individual modules may each have their own storage module. Morphisms such as these are within the scope of protection of the present disclosure.

FIG. 6 is a schematic diagram illustrating an exemplary processing circuit according to some embodiments of the present disclosure.

A processing circuit 600 in FIG. 6 may also realize the collecting and processing of physiological signals. The processing circuit 600 may achieve the purpose of saving space cost and reducing the hardware requirements by adopting a time-sharing multiplexing manner while ensuring the collection and processing of a plurality of signals. Specifically, as shown in FIG. 6, the processing circuit 600 may include at least two signal collection circuits (e.g., a first collection circuit 611, a second collection circuit 612, corresponding to the above-described first sub-circuit and the second sub-circuit), a switch circuit 620, a simulated circuit 630, and a control circuit 640. In some embodiments, the at least two signal collection circuits are configured to collect at least two physiological signals. The first collection circuit 611 and the second collection circuit 612 may have different input impedances. For example, the first collection circuit 611 has a large input impedance (e.g., an impedance larger than 1 MΩ) and the second collection circuit 612 has a small input impedance (e.g., an impedance in the order of 100 KΩ). The switch circuit 620 may be configured to control a conduction state between each signal collection circuit and the simulated circuit 630. For example, at a certain time point, the switch circuit 620 may conduct one signal collection circuit and the simulated circuit 630. Merely by way of example, at a certain time point, the switch circuit 620 may connect an electrode module with the first collection circuit 611 having the large input impedance, realizing the conduction of the first collection circuit 611 and the simulated circuit 630. The switch circuit 620 may cycle the conduction between each signal collection circuit and the simulated circuit 630 cyclically within a certain period of time. When the switch circuit 620 conducts a signal collection circuit and the simulated circuit 630, a signal collected by the signal collection circuit may be transmitted to the simulated circuit 630 for processing (e.g., noise reduction, amplification, etc.), and a processed signal is transmitted to the control circuit 640 for signal analysis. It should be understood that by setting the switch circuit 620 between a plurality of signal collection circuits and the simulated circuit 630, the same simulated circuit can process signals from different signal collection circuits at different time points, which can effectively reduce the complexity and cost of using multiple simulated circuits, and also reduce the count of channels for signal transmission between the simulated circuits and the control circuit. It should be understood that the switch circuit 620 and the simulated circuit 630 shown in FIG. 6 are only for illustrative purposes, and in actual use, the plurality of signal collection circuits and the control circuit 640 may also be connected through more than one switch. In some embodiments, the at least two signal collection circuits may include different resistors realizing different input impedances, and the switch circuit 620 may also be disposed between the electrode module and the resistors to control the connection between the electrode module and different resistors to realize signal collection circuits with different input impedances. The signal collection circuits with different input impedances may be further connected to the simulated circuit 630.

The control circuit 640 samples a signal processed by the simulated circuit 630. In some embodiments, a sampling frequency of the control circuit 640 is related to a count of the signal collection circuits, a control strategy for the switch circuit, and a target frequency of the physiological signal. For example, the control circuit 640 samples the signal of each circuit at a frequency no less than two times the target frequency of the signal. For example, for an EMG signal, assuming that a target frequency corresponding to the EMG signal is within 1,000 Hz, the control circuit may sample the EMG signal at a sampling frequency of 2,000 Hz. For the entire signal processing circuit, assuming that there are four signal collection circuits for collecting EMG signals, the control circuit 640 may provide a total sampling frequency of 8,000 Hz, which ensures that a sampling frequency for EMG signals of each circuit is 2000 Hz.

In some embodiments, the switch circuit 620 is configured to control the conduction of the at least two signal collection circuits with the simulated circuit 630, such that at a specific time a target signal collected by only a portion of the at least two signal collection circuits is transmitted to the simulated circuit 630. In some embodiments, the switch circuit 620 may include a switch chip having multiple channels and dual outputs, for example, a TMUX1209 switch chip, etc. In different embodiments, the control circuit 640 may control the switching of the switch circuit 620 based on different strategies. For example, to make the sampling data retain complete information of each target signal (i.e., the control circuit 640 may reconstruct each target signal based on the sampling data), the control circuit 640 may employ a full reconfiguration strategy to control the switching of the switch circuit 620. As another example, considering that the control circuit 640 may not be able to obtain valid sampling data during the rapid switching of switch channels, the control circuit 640 may use a splicing signal strategy to control the switching of the switch circuit 620.

The simulated circuit 630 is configured to process a physiological signal received therein. In some embodiments, since a raw physiological signal directly collected by the signal collection circuit has a very small amplitude and a large amount of noise, operations such as filtering, differential amplification, amplification, negative feedback noise cancellation, etc., may be performed on the raw physiological signal by the simulated circuit 630. In some embodiments, the simulated circuit 630 may include a differential amplifier configured to suppress common mode signals and amplify the physiological signal that the simulated circuit 630 receives. In some embodiments, the simulated circuit 630 may include a multi-stage amplification circuit for amplifying the physiological signal received therein. In some embodiments, the simulated circuit 630 may include a filter circuit for filtering the physiological signal received therein. In some embodiments, the simulated circuit 630 may include a right-leg drive circuit for extracting a common mode signal in the physiological signal received therein, reversely amplifying the common mode signal, and then feeding back to a signal source, which can mainly suppress the industrial frequency in the signal source. In some embodiments, the simulated circuit 630 may include a differential amplifier, a multi-stage amplifier, a filter circuit, and a right-leg drive circuit, or only one or more of them.

As described above, the control circuit 640 is used to receive the physiological signal processed by the simulated circuit 630 and sample the processed physiological signal. In some embodiments, the control circuit 640 includes a plurality of ADC channels, each of which may be configured to convert the physiological signal processed by the simulated circuit 630 into a digital signal for reading and processing. For example, the first collection circuit 611 and the second collection circuit 612 may have different input impedances, and physiological signals collected by the two circuits may include motion artifact signals of different proportions. The control circuit 640 may process the physiological signals based on motion artifact signals of different proportions to obtain a target physiological signal (i.e., a pure physiological signal). In some embodiments, the control circuit 640 may also be coupled to a display device to provide a display of a read digital signal, thereby visualizing the physiological signals. In some embodiments, based on the sampling, the control circuit 640 may read, store, process, analyze, etc., the physiological signals. Optionally, the control circuit 640 may also send a corresponding instruction based on sampled data.

It should be appreciated that the processing circuit 600 shown in FIG. 6 is for illustrative purposes only. In some embodiments, the processing circuit 600 may include one or more other components, or one or more components of the processing circuit 600 may be combined or omitted. In some embodiments, the count, location, etc., of the one or more components in the processing circuit 600 may be varied, which is not limited in the present disclosure.

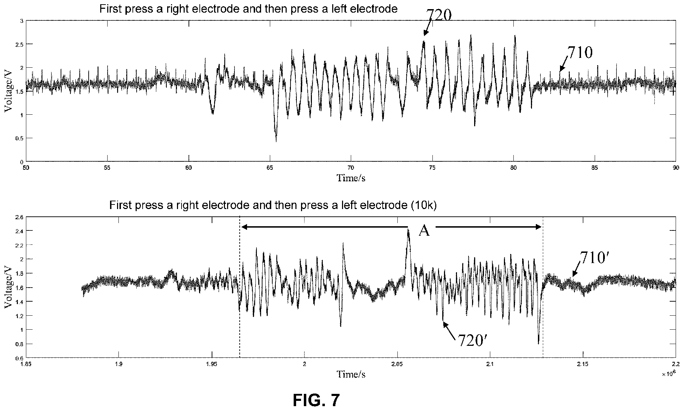

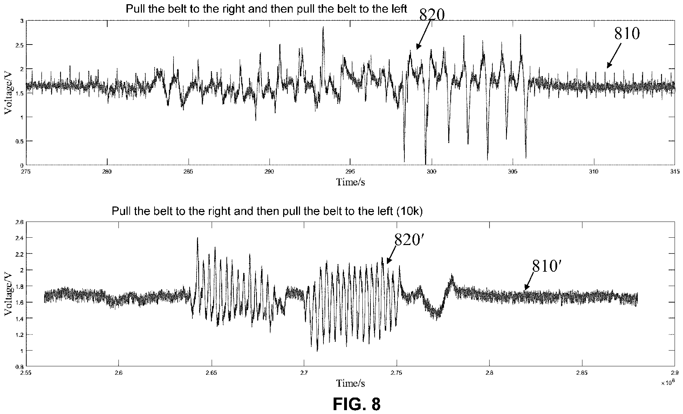

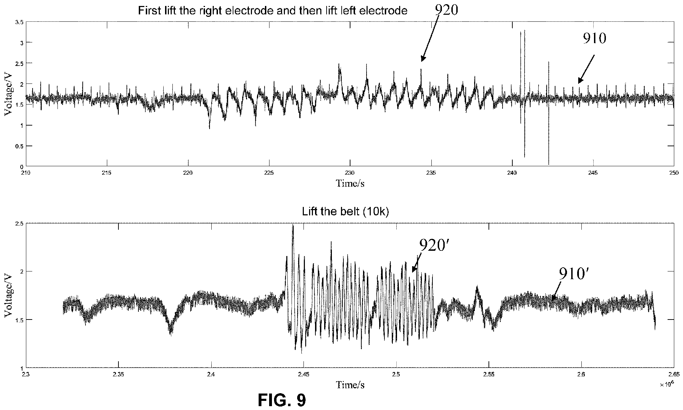

FIG.7 is a schematic diagram illustrating electrocardiographic signals including motion artifacts caused by a relative displacement of a horizontal action according to some embodiments of the present disclosure; FIG.8 is a schematic diagram illustrating electrocardiographic signals including motion artifacts caused by a relative displacement of a vertical action according to some embodiments of the present disclosure; and FIG. 9 is a schematic diagram illustrating electrocardiogram signals including motion artifacts caused by a compression according to some embodiments of the present disclosure.

In order to obtain a difference between the ECG signals (i.e., physiological signals collected in a time-sharing mode) collected by the circuit with the large input impedance and the circuit with the small input impedance (e.g., 10 KΩ), FIG. 7, FIG. 8, and FIG. 9 (in FIG. 7, FIG. 8, and FIG. 9, burrs denote the ECG signals or motion artifact signals, e.g., a burr 710, a burr 810, and a burr 910, a burr 710', a burr 810', and a burr 910', are burrs corresponding to the ECG signals; burrs corresponding to the motion artifact signals, e.g., a burr 720, a burr 820, a burr 920, a burr 720', a burr 820', and a burr 920', have peaks/valleys with greater amplitudes than the burrs corresponding to the ECG signals,; more descriptions regarding the burrs can be found in FIG. 4 and relevant descriptions thereof) shows comparisons of the three artificial motion artifact sources including a relative displacement caused by the horizontal action, a relative displacement caused by the vertical action, and compression, respectively. In FIG. 7, FIG. 8, and FIG. 9, the upper of the figure is an ECG signal collected by a circuit with a large input impedance, and the lower of the figure is an ECG signal collected by a circuit with a small input impedance. According to FIG. 7, FIG. 8, and FIG. 9, motion artifacts can be more clearly detected by the circuit with the small input impedance than by the circuit with the large input impedance.

FIG. 10A is a schematic diagram illustrating a processing circuit of a signal processing system according to some embodiments of the present disclosure. FIG. 10B is a schematic diagram illustrating a processing circuit of a signal processing system according to some embodiments of the present disclosure. FIG. 10C is a schematic diagram illustrating a processing circuit of a signal processing system according to some embodiments of the present disclosure.

In some embodiments, the processing circuit may include a switch and two signal processing sub-circuits, the switch is configured to control a group of electrodes to be connected to one of the two signal processing sub-circuits and disconnected from the other signal processing sub-circuit of the two signal processing sub-circuits in one time period among different time periods, and the two signal processing sub-circuits have different input impedances.

As shown in FIG. 10A, an electrode module 1010 includes two electrodes (a first electrode 1011 and a second electrode 1012), and the electrode module 1010 is connected to two signal processing sub-circuits via a switch 1020. For example, the switch 1020 may include at least one group of input ends and at least two groups of output ends (e.g., a first group of output ends 1031 and a second group of output ends 1032), and the switch 1020 is connected to the electrode module 1010 through one of the at least one group of input ends (e.g., including two input ends), and connected to the two signal processing sub-circuits through two groups of output ends. The two signal processing sub-circuits (a sub-circuit in which R4 is located is referred to as a first sub-circuit 1041, and a sub-circuit in which R5 is located is referred to as a second sub-circuit 1042) have different input impedances (i.e., R4 and R5), and each of the two sub-circuits is connected to the switch 1020 such that the switch 1020 may control the disconnection/connection between the electrode module 1010 and one of the sub-circuits. The switch 1020 includes a time-sharing multiplexing switch that may be configured to: in one time period among the different time periods, control the on/off of a group of output ends of the switch 1020 to control the electrodes to be connected to one of the two signal processing sub-circuits and disconnecting from the other signal processing sub-circuit of the two signal processing sub-circuits. For example, in a first time period, the switch 1020 turns off the first group of output ends 1031 and turns on the second group of output ends 1032, such that the electrode module 1010 is connected to the second sub-circuit 1042, and the input impedance is R5. As another example, in a second time period, the switch 1020 turns off the second group of output ends 1032 and turns on the first group of output ends 1031, such that the electrode module 1010 is connected to the first sub-circuit 1041, and the input impedance is R4. It should be understood that the circuit shown in FIG. 10A is for illustration purposes only. In some embodiments, as shown in FIG. 10B, a group of input ends and/or output ends of the switch 1020 may be represented by a single connection line, which is not limited in the present disclosure. In some embodiments, as shown in FIG. 10C, the switch 1020 may be provided at a reference ground connected to the processing circuit (e.g., the first sub-circuit 1041 and the second sub-circuit 1042) to control the disconnection/connection between the electrode module 1010 and one of the sub-circuits. Moreover, in conjunction with FIG. 10A, the input impedance described herein refers to an impedance between the input end of the processing circuit (e.g., the first sub-circuit 1041 or the second sub-circuit 1042) to the reference ground.

In some embodiments, a ratio of the input impedances of the two signal processing sub-circuits (i.e., a ratio of the impedance of R4 to the impedance of R5) is not less than 10.

In some embodiments, each of the two signal processing sub-circuits may include a differential amplifier configured to differentially amplify signals collected by the electrodes.

The differential amplifier is an electronic device that may amplify a difference between voltages of two input ends with a fixed gain, which may amplify a difference between the signals collected by the electrodes, and increase an output value of collected signals, thereby enhancing the signals.

In some embodiments of the present disclosure, the signals collected by the processing circuit always originate from the same group of electrodes, while a time-sharing multiplexing manner is used to collect the signals from the same group of electrodes using different input impedances. In such cases, collection errors caused by using different impedances to connect different groups of electrodes, respectively can be avoided, and measurement accuracy can be improved. Additionally, by adding a differential amplifier to the circuit, the collected signals can be enhanced, thereby facilitating further analysis and calculation of the signals.

FIG. 11 is a schematic diagram illustrating another processing circuit of a signal processing system according to some embodiments of the present disclosure.

In some embodiments, the processing circuit may include a switch and a resistor connected in parallel with an input end of the processing circuit. The switch may control the resistor to be connected in parallel with or disconnected from the input end in different time periods.

As shown in FIG. 11, an electrode module 1110 includes two electrodes (a first electrode 1111 and a second electrode 1112), and the electrode module 1110 is connected to a third sub-circuit 1141. The third sub-circuit 1141 is a sub-circuit that includes only an input impedance R6. For example, one of the first electrode 1111 and the second electrode 1112 may be connected to one of the two input ends of the third sub-circuit 1141, and the other one of the first electrode 1111 and the second electrode 1112 may be connected to the other one of the two input ends of the third sub-circuit 1141. Further, the processing circuit also includes a switch 1120 and a resistor R7, the resistor R7 being connected in series with the switch 1120, and the switch 1120 being configured to control the resistor R7 to form a parallel circuit with the input impedance R6 or disconnect the resistor R7 and the input impedance R6. For example, when the switch 1120 is turned off, the input impedance R6 is not connected in parallel with the resistor R7, and the processing circuit actually uses the third sub-circuit 1141 to collect the signal; when the switch 1120 is turned on, the resistor R7 is connected in parallel with the input end of the third sub-circuit 1141 (or with the input impedance R6), and the processing circuit actually uses a fourth sub-circuit 1142 to collect a signal. The fourth sub-circuit 1142 is a sub-circuit formed by the parallel connection of the input impedance R6 and the resistor R7. The switch 1120 may include a time-sharing multiplexing switch that may be configured to: in one time period among different time periods, control the disconnection/connection between the processing circuit and the resistor R7, thereby controlling the electrodes to be connected to one of the two signal processing sub-circuits. For example, in a first time period, the switch 1120 is turned off, and the processing circuit actually uses the third sub-circuit 1141 to collect the signal. As another example, in a second time period, the switch 1120 is turned on, the processing circuit actually uses the fourth sub-circuit 1142 to collect the signal, and an actual input impedance of the processing circuit is impedances of R6 and R7 connected in parallel. It will be appreciated that the third sub-circuit 1141 and the fourth sub-circuit 1142 in this embodiment may share some of the circuit. In some embodiments, the switch 1120 may also be provided at a reference ground connected to the processing circuit (e.g., the third sub-circuit 1141) to adjust the input impedance of the processing circuit in a time-sharing mode.

In some embodiments, a ratio of an input impedance of the processing circuit when the switch is turned off to the resistor is not less than 10. In a circuit structure shown in FIG. 11, R6 may be not less than 1 MΩ, and R7 may be not greater than 100 KΩ. In such cases, the input impedance of the processing circuit is R6 when the switch of the processing circuit is turned off, and when the switch of the processing circuit is turned on, the resistor R7 is connected in parallel with R6, and the input impedance of the circuit is a total impedances in parallel (since the total impedances in parallel is smaller than the impedance of any of the sub-circuits, the total impedances in parallel is less than 100 K Ω). Therefore, the ratio of the input impedance of the processing circuit when the switch is turned off to the resistor is not less than 10 such that a ratio of the input impedance of the processing circuit when the switch is turned off to an input impedance of the processing circuit when the switch is turned on may be not less than 10, which achieves the purpose collecting a physiological signal based on a large input impedance and collecting the motion artifact signal based on a small input impedance.

In some embodiments of the present disclosure, the circuit structure described above enables the ECG signal to be sufficiently collected by the circuit using the large input impedance, whereas the ECG signal collected using the small input impedance may be small, and the motion artifact signal collected using the small input impedance is enhanced, thereby improving the processing accuracy. Additionally, the design of the circuit structure described above simplifies the circuit structure and reduces the count of electronic components, which facilitates manufacturing and reduces costs.

In some embodiments of the present disclosure, the signals collected by the processing circuit always originate from the same group of electrodes, while a time-sharing multiplexing manner is used to collect signals from the same group of electrodes using different input impedances. In such cases, the collected signals all originate from the same group of electrodes, which have better consistency and lower collection errors, thereby improving the collection accuracy. Additionally, the use of the switch to control the resistor connected in parallel to be disconnected from or connected to the circuit can simplify the circuit.

FIG. 12 is a schematic diagram illustrating a processing circuit of another signal processing system according to some embodiments of the present disclosure.

In some embodiments, the signal processing system may include two groups of electrodes (e.g., a group of electrodes 1210 and a group of electrodes 1220 in FIG. 12), each group of the two groups of electrodes being configured to fit the human body to collect physiological signals; and a processing circuit configured to read the physiological signals collected by each group of the groups of electrodes, respectively. The processing circuit has different input impedances when reading the physiological signals collected by the different groups of electrodes, and the physiological signals read by the processing circuit include motion artifact signals of different proportions. The processing circuit may process the physiological signals based on the motion artifact signals of different proportions.

In some embodiments, the processing circuit includes two sub-processing circuits, and each of the two sub-processing circuits is connected to one group of the two groups of electrodes. The two sub-processing circuits have different input impedances. In the processing circuit shown in FIG. 12, the group of electrodes 1210 includes two electrodes (a first electrode 1211 and a second electrode 1212), and the group of electrodes 1220 includes two electrodes (a third electrode 1221 and a fourth electrode 1222); the group of electrodes 1210 are connected to a sub-processing circuit 1230, and the group of electrodes 1220 are connected to a sub-processing circuit 1240. The sub-processing circuit 1230 has an input impedance of R8 and the sub-processing circuit 1240 has an input impedance of R9. The sub-processing circuit 1230 and the sub-processing circuit 1240 may collect signals from the group of electrodes 1210 and the group of electrodes 1220, respectively, for subsequent processing.

In some embodiments, in the processing circuit shown in FIG. 12, a ratio of the largest input impedance (e.g., R8) in the different input impedances to the smallest input impedance (e.g., R9) in the different input impedances is not less than 10. For example, a ratio of the input impedance R8 to the input impedance R9 is 20.

In some embodiments, in the processing circuit shown in FIG. 12, the largest input impedance (e.g., R8) in the different input impedances is not less than 1 MΩ, and the smallest input impedance (e.g., R9) in the different input impedances is not greater than 100 KΩ. For example, the input impedance R8 is 2 MΩ and the input impedance R9 is 10 KΩ.

In some embodiments, the processing circuit may obtain a pure physiological signal by performing a filtering operation on the motion artifact signals based on a correspondence of the physiological signals including the motion artifact signals of different proportions, and obtain a target physiological signal by performing an enhancement operation on the pure physiological signal. For example, the processing circuit may identify the motion artifact signals based on the physiological signal read at the smallest input impedance, take signals with high correlation with the motion artifact signals as the motion artifact signals in the physiological signal read at the largest impedance, and filter the motion artifact signals from the physiological signal read at the largest impedance. Merely by way of example, when motion artifacts are caused by factors such as motion (e.g., running, jumping, etc.), the physiological signal read at the smallest input impedance may have obvious fluctuations (as shown by range A in FIG. 6), and the processing circuit may identify the signals having obvious fluctuations and take the identified signals as the motion artifact signals. Further, the processing circuit may determine a correspondence between the physiological signal read at the largest input impedance and the motion artifact signals, obtain a pure physiological signal by performing a filtering operation on the motion artifact signals in the physiological signals, and/or obtain a target physiological signal by performing an enhancement operation on the pure physiological signal.

When the processing circuit shown in FIG. 12 does not use a time-multiplexing scheme but connects two circuits (e.g., the sub-processing circuit 1230 and the sub-processing circuit 1240) to two groups of electrodes (e.g., the group of electrodes 1210 and the group of electrodes 1220), respectively, the motion artifact signals measured by the two groups of electrodes should be as same as possible. Therefore, the consistency of the two groups of electrodes is required to be high. For example, the two groups of electrodes are sufficiently close to each other, sufficiently similar in affixation states, etc., so that the motion artifact signals collected using the smallest input impedance may be used to characterize the motion artifact signals in the physiological signal collected using the largest input impedance, thereby filtering the motion artifact signals. Therefore, a distance between two electrodes in different electrode modules needs to be specified. Each electrode module includes two electrodes, and to improve the consistency of the motion artifact signals collected using the smallest input impedance with the motion artifact signals in the physiological signal collected using the largest impedance, as shown in FIG. 12, when the two groups of electrodes are set adjacent to each other, a minimum distance between two electrodes in different groups is less than a distance threshold. The minimum distance refers to a distance between two closest electrodes in different groups of electrodes (e.g., a distance between the midpoints of two closest edges, an average of distances between multiple points on the two closest edges, etc.). The distance threshold may be preset, for example, the minimum distance between the two electrodes in different groups may be less than 5 cm. As another example, the minimum distance between the two electrodes in the different groups may be less than 3 cm. As a further example, the minimum distance between the two electrodes in the different groups may be less than 1 cm. In some embodiments, the two groups of electrodes may be provided in a manner that is not limited to the adjacent arrangement as illustrated in FIG. 12. For example, four electrodes in the two groups of electrodes may be set side-by-side, and two electrodes in one group of electrodes are located on either side of the other group of electrodes. In other words, one group of electrodes may enclose the other group of electrodes, and the two groups of electrodes may partially overlap. For example, a center (e.g., a centroid) of one group of electrodes coincides or approximately coincides with a center of the other group of electrodes. For example, a distance between the center of one group of electrodes and the center of the other group of electrodes may be less than 5 cm. As another example, the distance between the center of one group of electrodes and the center of the other group of electrodes may be less than 3 cm. As another example, the distance between the center of one group of electrodes and the center of the other group of electrodes may be less than 1 cm.

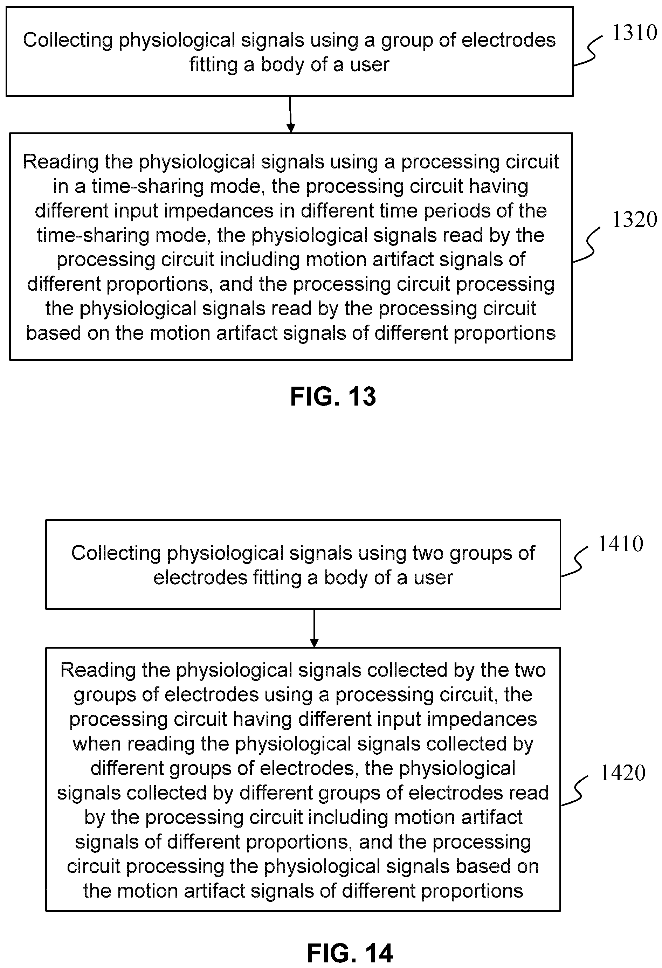

FIG. 13 is a flowchart illustrating an exemplary signal processing process according to the present invention.

In 1310, physiological signals are collected in different time periods using a group of electrodes that fit the body of a user. Descriptions regarding the group of electrodes and the physiological signals can be found in FIG. 2 and relevant descriptions thereof.

In 1320, the physiological signals are read by a processing circuit in a time-sharing mode. The processing circuit has different input impedances in the different time periods of the time-sharing mode, and the physiological signals read by the processing circuit include motion artifact signals of different proportions. The processing circuit is configured to process the physiological signals read by the processing circuit based on the motion artifact signals of different proportions. More descriptions regarding reading the physiological signals can be found in FIG. 2, FIG. 10 A, FIG. 10 B, FIG. 11, and relevant descriptions thereof.

In the invention, the different input impedances include two input impedances, where a ratio of the largest input impedance in the two input impedances to the smallest input impedance in the two input impedances is not less than 10.

In some embodiments, the largest input impedance in the two input impedances is not less than 1 MΩ, and the smallest input impedance in the two input impedances is not greater than 100 KΩ. For example, the largest input impedance is 2 MΩ, and the smallest input impedance is 10 KΩ.

In some embodiments, in one time period of the different time periods, a switch may be used to control the group of electrodes to be connected to one of the two signal processing sub-circuits and disconnected from the other signal processing sub-circuit, and the two signal processing sub-circuits have different input impedances. More descriptions can be found in FIG. 10A, FIG. 10B, and relevant descriptions thereof.

In some embodiments, each of the two signal processing sub-circuits may include a differential amplifier, by which the signals collected by the group of electrodes are differentially amplified.

In some embodiments, the processing circuit includes a switch and a resistor connected in parallel with an input end of the processing circuit. The switch may control the resistor to be connected in parallel with or disconnected from the input end in different time periods. More descriptions can be found in FIG. 11 and relevant descriptions thereof.

In some embodiments, a ratio of the input impedance of the processing circuit when the switch is turned off to the resistor is not less than 10.

In some embodiments, the processing circuit may obtain a pure physiological signal by performing a filtering operation on the motion artifact signals from the physiological signals based on a correspondence of the physiological signals including the motion artifact signals of different proportions, and obtain a target physiological signal by performing an enhancement operation on the pure physiological signal. For example, the processing circuit may identify the motion artifact signals based on the physiological signal read at the smallest input impedance, and take a signal with a high correlation with the motion artifact signal in the physiological signal read at the large impedance as the motion artifact signal to be filtered out physiological signal read at the largest impedance. Merely by way of example, when motion artifacts are caused by factors such as motion (e.g., running, jumping, etc.), the physiological signal read at the smallest input impedance may have obvious fluctuations (as shown by range A in FIG. 6), and the processing circuit may identify the signal having the obvious fluctuations and take the signal as the motion artifact signal. Further, the processing circuit may determine a correspondence between the physiological signal read at the largest input impedance and the motion artifact signal, obtain a pure physiological signal by performing a filtering operation on the motion artifact signal from the physiological signals, and/or obtain a target physiological signal by performing the enhancement operation on the pure physiological signal.

FIG. 14 is a flowchart illustrating another signal processing process according to the present invention.

In 1410, physiological signals is collected by two groups of electrodes fitting to the body of a user. More descriptions regarding collecting the physiological signals can be found in FIG. 2, FIG. 12, and relevant descriptions thereof.

In 1420, the physiological signals collected by each group of electrodes are read by the processing circuit. The processing circuit has different input impedances when reading the physiological signals collected by the different groups of electrodes, and the physiological signals read by the processing circuit include motion artifact signals of different proportions. The processing circuit processes the physiological signals based on the motion artifact signals of different proportions. More descriptions regarding the processing circuit can be found in FIG. 12 and relevant descriptions thereof.

In some embodiments, each group of electrodes includes two electrodes, and a minimum distance between two electrodes in different groups is less than a distance threshold. The distance threshold may be preset, for example, the two groups of electrodes may be adjacent to each other, and the minimum distance between the two electrodes in different groups may be less than 5 cm, 3 cm, 1 cm, or the like. As another example, one group of electrodes in the two groups of electrodes may enclose the other group of electrodes in the two groups of electrodes, and a distance between a center of one group of electrodes and a center of the other group of electrodes may be less than 5 cm, 3 cm, 1 cm, etc.

In the invention, a ratio of the largest input impedance in the different input impedances to the smallest input impedance in the different input impedances is not less than 10.

In some embodiments, the largest input impedance is not less than 1 M$\Omega$, and the smallest input impedance is not greater than 100 K$\Omega$. For example, the largest input impedance is 2 M$\Omega$ and the smallest input impedance is 10 K$\Omega$.

In some embodiments, the processing circuit includes two sub-processing circuits respectively connected to one of the two groups of electrodes, and the two sub-processing circuits have different input impedances. More descriptions regarding the processing circuit can be found in FIG. 12 and relevant descriptions thereof.

In some embodiments, the processing circuit may obtain a pure physiological signal by performing a filtering operation on the motion artifact signals based on a correspondence of the physiological signals including the motion artifact signals of different proportions, and obtain a target physiological signal by performing an enhancement operation on the pure physiological signal. For example, the processing circuit may identify the motion artifact signal based on the physiological signal read at the smallest impedance, and a signal with a high correlation with the motion artifact signal in the physiological signal read at the largest impedance may be regarded as the motion artifact signal to be filtered out physiological signal read at the largest impedance. Merely by way of example, when motion artifacts are caused by factors such as motion (e.g., running, jumping, etc.), the physiological signal read at the smallest input impedance may have obvious fluctuations (as shown by range A in FIG. 6), and the processing circuit may identify a signal having the obvious fluctuations and take the signal as the motion artifact signals. Further, the processing circuit may determine a correspondence between the physiological signal read at the largest input impedance and the motion artifact signal, obtain a pure physiological signal by performing a filtering operation on the motion artifact signals from the physiological signals, and/or obtain a target physiological signal by performing an enhancement operation on the pure physiological signal.

The basic concepts have been described above, and it is apparent to those skilled in the art that the foregoing detailed disclosure serves only as an example and does not constitute a limitation of the present disclosure. While not expressly stated herein, a person skilled in the art may make various modifications, improvements, and amendments to the present disclosure.

Also, the present disclosure uses specific words to describe embodiments of the present disclosure. Such as "an embodiment," "one embodiment," and/or "some embodiment" means a feature, structure, or characteristic associated with at least one embodiment of the present disclosure. Accordingly, it should be emphasized and noted that "an embodiment" or "one embodiment," or "an alternative embodiment" in different locations in the present disclosure do not necessarily refer to the same embodiment. In addition, certain features, structures, or characteristics in one or more embodiments of the present disclosure may be suitably combined.

Furthermore, unless expressly stated in the claims, the order of the processing elements and sequences, the use of numerical letters, or the use of other names as described in the present disclosure are not intended to qualify the order of the processes and methods of the present disclosure. While some embodiments of the invention that are currently considered useful are discussed in the foregoing disclosure by way of various examples, it is to be understood that such details serve only illustrative purposes and that additional claims are not limited to the disclosed embodiments, rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the presentation of the present disclosure, and thereby aid in the understanding of one or more embodiments of the invention, the foregoing descriptions of embodiments of the present disclosure sometimes group multiple features together in a single embodiment, accompanying drawings, or in a description thereof. However, this method of disclosure does not imply that the objects of the present disclosure require more features than those mentioned in the claims. Rather, claimed subject matter may lie in less than all features of a single foregoing disclosed embodiment.

Some embodiments use numbers to describe the number of components, attributes, and it should be understood that such numbers used in the description of the embodiments are modified in some examples by the modifiers "about", "approximately", or "substantially". Unless otherwise noted, the terms "about," "approximately," or "substantially" indicates that a $\pm 20\%$ variation in the stated number is allowed. Correspondingly, in some embodiments, the numerical parameters used in the present disclosure and claims are approximations, which approximations are subject to change depending on the desired characteristics of individual embodiments. In some embodiments, the numerical parameters should take into account the specified number of valid digits and employ general place-keeping. While the numerical domains and parameters used to confirm the breadth of their ranges in some embodiments of the present disclosure are approximations, in specific embodiments, such values are set to be as precise as practicable.

**Claims**

1. A signal processing system (200), comprising:

   a group of electrodes (210) configured to fit a body of a user to collect physiological signals of the user in different time periods; and
   a processing circuit (220) configured to read the physiological signals in a time-sharing mode, wherein

   the processing circuit (220) has different input impedances in the different time periods of the time-sharing mode, wherein the processing circuit (220) has a first input impedance in a first time period and a second input impedance in a second time period, and a ratio of the first input impedance to the second input impedance is not less than 10, wherein the first input impedance is configured to enhance a proportion of pure physiological signals in the collected physiological signals and the second input impedance is configured to amplify motion artifact signals, in the collected physiological signals, caused by skin-electrode interface variations, so that the physiological signals read by the processing circuit (220) in the first time period and the second time period include motion artifact signals of different proportions, and
   the processing circuit (220) is configured to process the physiological signals read by the processing circuit (220) based on the motion artifact signals of different proportions.

2. The signal processing system (200) of claim 1, wherein the first input impedance is not less than 1 MΩ, and the second input impedance is not greater than 100 KΩ.

3. The signal processing system (200) of claim 1 or claim 2, wherein

   the processing circuit (220) includes a switch (1020) and at least two signal processing sub-circuits, and
   in one of the different time periods, the switch (1020) is configured to control the group of electrodes (210) to be connected to one of the at least two signal processing sub-circuits and disconnected from the other signal processing sub-circuit of the at least two signal processing sub-circuits, the at least two signal processing sub-circuits having different input impedances.

4. The signal processing system (200) of claim 3, wherein each of the at least two signal processing sub-circuits includes a differential amplifier configured to differentially amplify signals collected by the group of electrodes (210).

5. The signal processing system (200) of claim 1 or claim 2, wherein the processing circuit (220) includes a switch (1120) and a resistor (R7) connected in parallel with an input end of the processing circuit (220), the switch (1120) being configured to control the resistor (R7) to be connected in parallel with or disconnected from the input end in the different time periods.

6. The signal processing system (200) of claim 5, wherein when the switch (1120) of the processing circuit (220) is disconnected, a ratio of the input impedance of the processing circuit (220) to the resistor (R7) is not less than 10.

7. The signal processing system (200) of any one of claims 1-6, wherein the processing circuit (220) is configured to process the physiological signals read by the processing circuit (220) based on the motion artifact signals of different proportions by:

   obtaining a pure physiological signal by performing a filtering operation on the motion artifact signals from the physiological signals based on a correspondence of the physiological signals including the motion artifact signals of different proportions, and/or
   obtaining a target physiological signal by performing an enhancement operation on the pure physiological signal.

8. The signal processing system (200) of claim 1, wherein the second input impedance is in a same order as a contact impedance (R2) generated when a signal propagates through different media at the point where the group of electrodes (210) touch skin of the body of the user.

9. The signal processing system (200) of claim 8, wherein a ratio of the second input impedance to the contact impedance (R2) is in a range of 0.1-10.

10. The signal processing system (200) of claim 1, wherein the time-sharing mode is achieved by reading physiological signals of a plurality of channels in a time-sharing multiplexing manner using a spliced signal strategy, the spliced signal strategy referring to sampling a physiological signal of each channel by slow switching of a time-sharing multiplexing switch, the slow switching of the time-sharing multiplexing switch referring to that the time-sharing multiplexing switch switches at a frequency that is less than a frequency of the physiological signal and greater than an action frequency of the user when the user is in motion.

11. The signal processing system (200) of claim 1, wherein the processing circuit (220) includes a simulated circuit (630) configured to process the physiological signals, the simulated circuit (630) includes a right-leg drive circuit for extracting a common mode signal in the physiological signals, reversely amplifying the common mode signal, and then feeding back to a signal source.

12. A signal processing system (200), comprising:

   two groups of electrodes (1210, 1220), each group of electrodes in the two groups of electrodes (1210, 1220) being configured to fit a body of a user to collect physiological signals; and
   a processing circuit (220) configured to read the physiological signals collected by the two groups of electrodes (1210, 1220), one of the two groups of electrodes (1210, 1220) has a first input impedance, the other of the two groups of electrodes (1210, 1220) has a second input impedance, and a ratio of the first input impedance to the second input impedance is not less than 10, wherein the first input impedance is configured to enhance a proportion of pure physiological signals in the collected physiological signals and the second input impedance is configured to amplify motion artifact signals, in the collected physiological signals, caused by skin-electrode interface variations, so that the physiological signals read by the processing circuit (220) include motion artifact signals of different proportions, and
   the processing circuit (220) is configured to process the physiological signals collected by the two groups of electrodes (1210, 1220) based on the motion artifact signals of different proportions.

13. The signal processing system (200) of claim 12, wherein each group of electrodes includes two electrodes, and a minimum distance between the two electrodes in different groups of the two groups of electrodes (1210, 1220) is less than 5 cm.

14. The signal processing system (200) of claim 12 or 13, wherein the processing circuit (220) includes at least two processing sub-circuits, configured to connect to each of the two groups of electrodes (1210, 1220), respectively, the at least two processing sub-circuits having different input impedances.

15. A signal processing method, comprising:

   collecting physiological signals through a group of electrodes (210) that fit a body of a user; and reading the physiological signals through a processing circuit (220) in a time-sharing mode, wherein

the processing circuit (220) has different input impedances in different time periods of the time-sharing mode, wherein the processing circuit (220) has a first input impedance in a first time period and a second input impedance in a second time period, and a ratio of the first input impedance to the second input impedance is not less than 10, wherein the first input impedance is configured to enhance a proportion of pure physiological signals in the collected physiological signals and the second input impedance is configured to amplify motion artifact signals, in the collected physiological signals, caused by skin-electrode interface variations, so that the physiological signals read by the processing circuit (220) in the first time period and the second time period include motion artifact signals of different proportions, and

the processing circuit (220) is configured to process the physiological signals read by the processing circuit (220) based on the motion artifact signals of different proportions.

**Patentansprüche**

1. Signalverarbeitungssystem (200), umfassend:

eine Gruppe von Elektroden (210), die so konfiguriert sind, dass sie an einen Körper eines Benutzers angepasst werden können, um physiologische Signale des Benutzers in verschiedenen Zeitabschnitten zu sammeln; und eine Verarbeitungsschaltung (220), die so konfiguriert ist, dass sie die physiologischen Signale im Zeitmultiplexbetrieb liest, wobei

die Verarbeitungsschaltung (220) in den verschiedenen Zeitabschnitten des Zeitmultiplexbetriebs unterschiedliche Eingangsimpedanzen aufweist, wobei die Verarbeitungsschaltung (220) in einem ersten Zeitabschnitt eine erste Eingangsimpedanz aufweist und in einem zweiten Zeitabschnitt eine zweite Eingangsimpedanz aufweist und ein Verhältnis der ersten Eingangsimpedanz zur zweiten Eingangsimpedanz nicht weniger als 10 beträgt, wobei die erste Eingangsimpedanz so konfiguriert ist, dass sie einen Abschnitt von rein physiologischen Signalen in den gesammelten physiologischen Signalen verstärkt, und die zweite Eingangsimpedanz so konfiguriert ist, dass sie Bewegungsartefaktsignale in den gesammelten physiologischen Signalen verstärkt, die durch Variationen an der Haut-Elektroden-Grenzfläche verursacht werden, sodass die von der Verarbeitungsschaltung (220) im ersten Zeitabschnitt und zweiten Zeitabschnitt gelesenen physiologischen Signale Bewegungsartefaktsignale unterschiedlicher Anteile beinhalten, und die Verarbeitungsschaltung (220) so konfiguriert ist, dass sie die von der Verarbeitungsschaltung (220) gelesenen physiologischen Signale basierend auf den Bewegungsartefaktsignalen unterschiedlicher Anteile verarbeitet.

2. Signalverarbeitungssystem (200) nach Anspruch 1, wobei die erste Eingangsimpedanz nicht weniger als 1 MΩ und die zweite Eingangsimpedanz nicht größer als 100 kΩ ist.

3. Signalverarbeitungssystem (200) nach Anspruch 1 oder Anspruch 2, wobei

die Verarbeitungsschaltung (220) einen Schalter (1020) und mindestens zwei Signalverarbeitungs-Unterschaltungen umfasst und in einem der verschiedenen Zeitabschnitte der Schalter (1020) so konfiguriert ist, dass er die Gruppe von Elektroden (210) so steuert, dass sie mit einer der mindestens zwei Signalverarbeitungs-Unterschaltungen verbunden werden und von der anderen Signalverarbeitungs-Unterschaltung getrennt werden, wobei die mindestens zwei Signalverarbeitungs-Unterschaltungen unterschiedliche Eingangsimpedanzen aufweisen.

4. Signalverarbeitungssystem (200) nach Anspruch 3, wobei jede der mindestens zwei Signalverarbeitungs-Unterschaltungen einen Differenzverstärker beinhaltet, der so konfiguriert ist, dass er die von der Gruppe von Elektroden (210) gesammelten Signale differenziell verstärkt.

5. Signalverarbeitungssystem (200) nach Anspruch 1 oder 2, wobei die Verarbeitungsschaltung (220) einen Schalter (1120) und einen Widerstand (R7) beinhaltet, die parallel mit einem Eingangsende der Verarbeitungsschaltung (220) geschaltet sind, wobei der Schalter (1120) so konfiguriert ist, dass er den Widerstand (R7) so steuert, dass er in den verschiedenen Zeitabschnitten parallel mit dem Eingangsende verbunden oder davon getrennt wird.

**6.** Signalverarbeitungssystem (200) nach Anspruch 5, wobei, wenn der Schalter (1120) der Verarbeitungsschaltung (220) getrennt ist, ein Verhältnis der Eingangsimpedanz der Verarbeitungsschaltung (220) zum Widerstand (R7) nicht weniger als 10 ist.

**7.** Signalverarbeitungssystem (200) nach einem der Ansprüche 1-6, wobei die Verarbeitungsschaltung (220) so konfiguriert ist, dass sie die von der Verarbeitungsschaltung (220) gelesenen physiologischen Signale basierend auf den Bewegungsartefaktsignalen unterschiedlicher Anteile verarbeitet durch:

Erhalten eines reinen physiologischen Signals durch einen Filterbetrieb an den Bewegungsartefaktsignalen aus den physiologischen Signalen basierend auf einer Entsprechung der physiologischen Signale, die die Bewegungsartefaktsignale unterschiedlicher Anteile beinhalten, und/oder
Erhalten eines physiologischen Zielsignals durch Durchführen einer Verstärkungsoperation an dem reinen physiologischen Signal.

**8.** Signalverarbeitungssystem (200) nach Anspruch 1, wobei die zweite Eingangsimpedanz in einer gleichen Größenordnung liegt wie eine Kontaktimpedanz (R2), die erzeugt wird, wenn sich ein Signal durch verschiedene Medien an der Stelle ausbreitet, an der die Gruppe von Elektroden (210) die Haut des Körpers des Benutzers berührt.

**9.** Signalverarbeitungssystem (200) nach Anspruch 8, wobei das Verhältnis der zweiten Eingangsimpedanz zur Kontaktimpedanz (R2) im Bereich von 0,1-10 liegt.

**10.** Signalverarbeitungssystem (200) nach Anspruch 1, wobei der Zeitmultiplexbetrieb durch Lesen physiologischer Signale einer Vielzahl von Kanälen im Zeitmultiplexbetrieb unter Verwendung einer Spleißsignal-Strategie erreicht wird, wobei sich die Spleißsignal-Strategie auf Abtasten eines physiologischen Signals jedes Kanals durch langsames Schalten eines Zeitmultiplex-Schalters bezieht, wobei sich das langsame Schalten des Zeitmultiplex-Schalters darauf bezieht, dass der Zeitmultiplex-Schalter mit einer Frequenz schaltet, die kleiner ist als eine Frequenz des physiologischen Signals und größer ist als eine Aktionsfrequenz des Benutzers, wenn sich der Benutzer in Bewegung befindet.

**11.** Signalverarbeitungssystem (200) nach Anspruch 1, wobei die Verarbeitungsschaltung (220) eine simulierte Schaltung (630) beinhaltet, die so konfiguriert ist, dass sie die physiologischen Signale verarbeitet, wobei die simulierte Schaltung (630) eine Ansteuerschaltung für den rechten Schenkel zum Extrahieren eines Gleichtaktsignals aus den physiologischen Signalen beinhaltet, das Gleichtaktsignal umgekehrt verstärkt und es dann an eine Signalquelle zurückführt.

**12.** Signalverarbeitungssystem (200), umfassend:

zwei Gruppen von Elektroden (1210, 1220), wobei jede Gruppe von Elektroden in den zwei Gruppen von Elektroden (1210, 1220) so konfiguriert ist, dass sie an einen Körper eines Benutzers angepasst werden kann, um physiologische Signale zu sammeln; und
eine Verarbeitungsschaltung (220), die so konfiguriert ist, dass sie die von zwei Gruppen von Elektroden (1210, 1220) gesammelten physiologischen Signale liest, wobei eine der zwei Gruppen von Elektroden (1210, 1220) eine erste Eingangsimpedanz aufweist, die andere der zwei Gruppen von Elektroden (1210, 1220) eine zweite Eingangsimpedanz aufweist und ein Verhältnis der ersten Eingangsimpedanz zur zweiten Eingangsimpedanz nicht weniger als 10 beträgt, wobei die erste Eingangsimpedanz so konfiguriert ist, dass sie einen Abschnitt rein physiologischer Signale in den gesammelten physiologischen Signalen verstärkt, und die zweite Eingangsimpedanz so konfiguriert ist, dass sie Bewegungsartefaktsignale in den gesammelten physiologischen Signalen verstärkt, die durch Variationen an der Haut-Elektroden-Grenzfläche verursacht werden, sodass die von der Verarbeitungsschaltung (220) gelesenen physiologischen Signale Bewegungsartefaktsignale unterschiedlicher Anteile beinhalten, und
die Verarbeitungsschaltung (220) so konfiguriert ist, dass sie die von den zwei Gruppen von Elektroden (1210, 1220) gesammelten physiologischen Signale basierend auf den Bewegungsartefaktsignalen unterschiedlicher Anteile verarbeitet.

**13.** Signalverarbeitungssystem (200) nach Anspruch 12, wobei jede Gruppe von Elektroden zwei Elektroden beinhaltet und ein Mindestabstand zwischen den zwei Elektroden in verschiedenen Gruppen der zwei Gruppen von Elektroden (1210, 1220) weniger als 5 cm beträgt.

**14.** Signalverarbeitungssystem (200) nach Anspruch 12 oder 13, wobei die Verarbeitungsschaltung (220) mindestens zwei Signalverarbeitungs-Unterschaltungen beinhaltet, die so konfiguriert sind, dass sie jeweils mit jeder der zwei Gruppen von Elektroden (1210, 1220) verbunden werden, wobei die mindestens zwei Signalverarbeitungs-Unterschaltungen unterschiedliche Eingangsimpedanzen aufweisen.

15. Signalverarbeitungsverfahren, umfassend:

Sammeln von physiologischen Signalen durch eine Gruppe von Elektroden (210), die an einen Körper eines Benutzers angepasst werden; und Lesen der physiologischen Signale durch eine Verarbeitungsschaltung (220) in einem Zeitmultiplexbetrieb, wobei

die Verarbeitungsschaltung (220) in verschiedenen Zeitabschnitten des Zeitmultiplexbetriebs unterschiedliche Eingangsimpedanzen aufweist, wobei die Verarbeitungsschaltung (220) in einem ersten Zeitabschnitt eine erste Eingangsimpedanz aufweist und in einem zweiten Zeitabschnitt eine zweite Eingangsimpedanz aufweist und ein Verhältnis der ersten Eingangsimpedanz zur zweiten Eingangsimpedanz nicht weniger als 10 beträgt, wobei die erste Eingangsimpedanz so konfiguriert ist, dass sie einen Abschnitt von rein physiologischen Signalen in den gesammelten physiologischen Signalen verstärkt, und die zweite Eingangsimpedanz so konfiguriert ist, dass sie Bewegungsartefaktsignale in den gesammelten physiologischen Signalen verstärkt, die durch Variationen an der Haut-Elektroden-Grenzfläche verursacht werden, sodass die von der Verarbeitungsschaltung (220) im ersten Zeitabschnitt und zweiten Zeitabschnitt gelesenen physiologischen Signale Bewegungsartefaktsignale unterschiedlicher Anteile beinhalten, und die Verarbeitungsschaltung (220) so konfiguriert ist, dass sie die von der Verarbeitungsschaltung (220) gelesenen physiologischen Signale basierend auf den Bewegungsartefaktsignalen unterschiedlicher Anteile verarbeitet.

**Revendications**

1. Système de traitement du signal (200), comprenant :

un groupe d'électrodes (210) configurées pour s'adapter au corps d'un utilisateur afin de collecter des signaux physiologiques de l'utilisateur dans différentes périodes de temps ; et un circuit de traitement (220) configuré pour lire les signaux physiologiques en mode de partage de temps, dans lequel

le circuit de traitement (220) présente des impédances d'entrée différentes dans les différentes périodes du mode de partage de temps, dans lequel le circuit de traitement (220) présente une première impédance d'entrée dans une première période de temps et une seconde impédance d'entrée dans une seconde période de temps, et un rapport entre la première impédance d'entrée et la seconde impédance d'entrée n'est pas inférieure à 10, dans lequel la première impédance d'entrée est configurée pour amplifier une proportion de signaux physiologiques purs dans les signaux physiologiques collectés et la seconde impédance d'entrée est configurée pour amplifier des signaux d'artefacts de mouvement, dans les signaux physiologiques collectés, dus à des variations de l'interface peau-électrode, de sorte que les signaux physiologiques lus par le circuit de traitement (220) dans la première période de temps et la seconde période de temps incluent des signaux d'artefacts de mouvement de proportions différentes, et le circuit de traitement (220) est configuré pour traiter les signaux physiologiques lus par le circuit de traitement (220) sur la base des signaux d'artefacts de mouvement de proportions différentes.

2. Système de traitement du signal (200) selon la revendication 1, dans lequel la première impédance d'entrée n'est pas inférieure à 1 M$\Omega$ et la seconde impédance d'entrée n'est pas supérieure à 100 K$\Omega$.

3. Système de traitement du signal (200) selon la revendication 1 ou la revendication 2, dans lequel

le circuit de traitement (220) inclut un commutateur (1020) et au moins deux sous-circuits de traitement du signal, et dans l'une des différentes périodes de temps, le commutateur (1020) est configuré pour commander le groupe d'électrodes (210) à connecter à l'un des au moins deux sous-circuits de traitement du signal et à déconnecter de l'autre sous-circuit de traitement du signal des au moins deux sous-circuits de traitement du signal, les au moins deux sous-circuits de traitement du signal présentant des impédances d'entrée différentes.

4. Système de traitement du signal (200) selon la revendication 3, dans lequel chacun des au moins deux sous-circuits de traitement du signal inclut un amplificateur différentiel configuré pour amplifier de manière différentielle des signaux collectés par le groupe d'électrodes (210).

**5.** Système de traitement du signal (200) selon la revendication 1 ou la revendication 2, dans lequel le circuit de traitement (220) inclut un commutateur (1120) et une résistance (R7) connectés en parallèle avec une extrémité d'entrée du circuit de traitement (220), le commutateur (1120) étant configuré pour commander la résistance (R7) afin qu'elle soit connectée en parallèle avec ou déconnectée de l'extrémité d'entrée dans les différentes périodes de temps.

**6.** Système de traitement du signal (200) selon la revendication 5, dans lequel lorsque l'interrupteur (1120) du circuit de traitement (220) est déconnecté, le rapport entre l'impédance d'entrée du circuit de traitement (220) et la résistance (R7) n'est pas inférieur à 10.

**7.** Système de traitement du signal (200) selon l'une quelconque des revendications 1-6, dans lequel le circuit de traitement (220) est configuré pour traiter les signaux physiologiques lus par le circuit de traitement (220) en fonction des signaux d'artefacts de mouvement de proportions différentes par :

l'obtention d'un signal physiologique pur en effectuant une opération de filtrage sur les signaux d'artefacts de mouvement à partir des signaux physiologiques sur la base d'une correspondance des signaux physiologiques incluant les signaux d'artefacts de mouvement de proportions différentes, et/ou
l'obtention d'un signal physiologique cible en effectuant une opération d'amélioration sur le signal physiologique pur.

**8.** Système de traitement du signal (200) selon la revendication 1, dans lequel la seconde impédance d'entrée est d'un même ordre qu'une impédance de contact (R2) générée lorsqu'un signal se propage à travers différents milieux au point où le groupe d'électrodes (210) touche la peau du corps de l'utilisateur.

**9.** Système de traitement du signal (200) selon la revendication 8, dans lequel un rapport entre la deuxième impédance d'entrée et l'impédance de contact (R2) est compris entre 0,1 et 10.

**10.** Système de traitement du signal (200) selon la revendication 1, dans lequel le mode de partage de temps est réalisé en lisant des signaux physiologiques d'une pluralité de canaux de manière multiplexée à partage de temps en utilisant une stratégie de signal épissé, la stratégie de signal épissé faisant référence à l'échantillonnage d'un signal physiologique de chaque canal par commutation lente d'un commutateur de multiplexage à partage de temps, la commutation lente du commutateur de multiplexage à partage de temps faisant référence au fait que le commutateur de multiplexage à partage de temps commute à une fréquence qui est inférieure à une fréquence du signal physiologique et supérieure à une fréquence d'action de l'utilisateur lorsque l'utilisateur est en mouvement.

**11.** Système de traitement du signal (200) selon la revendication 1, dans lequel le circuit de traitement (220) inclut un circuit simulé (630) configuré pour traiter les signaux physiologiques, le circuit simulé (630) inclut un circuit de commande de jambe droite pour extraire un signal de mode commun dans les signaux physiologiques, amplifier à l'inverse le signal de mode commun, puis le renvoyer à une source de signal.

**12.** Système de traitement du signal (200), comprenant :

deux groupes d'électrodes (1210, 1220), chaque groupe d'électrodes des deux groupes d'électrodes (1210, 1220) étant configuré pour s'adapter au corps d'un utilisateur afin de collecter des signaux physiologiques ; et
un circuit de traitement (220) configuré pour lire les signaux physiologiques collectés par les deux groupes d'électrodes (1210, 1220), un des deux groupes d'électrodes (1210, 1220) présente une première impédance d'entrée, l'autre des deux groupes d'électrodes (1210, 1220) présente une seconde impédance d'entrée, et un rapport entre le première impédance d'entrée et la seconde impédance d'entrée n'est pas inférieur à 10, dans lequel la première impédance d'entrée est configurée pour amplifier une proportion de signaux physiologiques purs dans les signaux physiologiques collectés et la seconde impédance d'entrée est configurée pour amplifier des signaux d'artefacts de mouvement, dans les signaux physiologiques collectés, dus à des variations de l'interface peau-électrode, de sorte que les signaux physiologiques lus par le circuit de traitement (220) incluent des signaux d'artefacts de mouvement de proportions différentes, et
le circuit de traitement (220) est configuré pour traiter les signaux physiologiques collectés par les deux groupes d'électrodes (1210, 1220) sur la base des signaux d'artefacts de mouvement de proportions différentes.

**13.** Système de traitement du signal (200) selon la revendication 12, dans lequel chaque groupe d'électrodes inclut deux électrodes, et une distance minimale entre les deux électrodes dans différents groupes des deux groupes d'électrodes (1210, 1220) est inférieure à 5 cm.

**14.** Système de traitement du signal (200) selon la revendication 12 ou 13, dans lequel le circuit de traitement (220) inclut au moins deux sous-circuits de traitement, configurés pour se connecter respectivement à chacun des deux groupes d'électrodes (1210, 1220), les au moins deux sous-circuits de traitement présentant des impédances d'entrée différentes.

**15.** Procédé de traitement du signal, comprenant :

la collecte de signaux physiologiques au moyen d'un groupe d'électrodes (210) qui s'adapte au corps de l'utilisateur ; et
la lecture des signaux physiologiques via un circuit de traitement (220) en mode partage de temps, dans lequel

le circuit de traitement (220) présente des impédances d'entrée différentes dans différentes périodes de temps du mode de partage de temps, dans lequel le circuit de traitement (220) présente une première impédance d'entrée dans une première période de temps et une seconde impédance d'entrée dans une seconde période de temps, et un rapport entre la première impédance d'entrée et la seconde impédance d'entrée n'est pas inférieur à 10, dans lequel la première impédance d'entrée est configurée pour amplifier une proportion de signaux physiologiques purs dans les signaux physiologiques collectés et la seconde impédance d'entrée est configurée pour amplifier des signaux d'artefacts de mouvement, dans les signaux physiologiques collectés, dus à des variations de l'interface peau-électrode, de sorte que les signaux physiologiques lus par le circuit de traitement (220) dans la première période de temps et la seconde période de temps incluent des signaux d'artefacts de mouvement de proportions différentes, et
le circuit de traitement (220) est configuré pour traiter les signaux physiologiques lus par le circuit de traitement (220) sur la base des signaux d'artefacts de mouvement de proportions différentes.

**100**

**FIG. 1**

## 200

### Electrodes
### 210

First electrode
211

Second
electrode 212

Processing circuit
220

# FIG. 2

**FIG. 3**

FIG. 4

**FIG. 5**

**600**

**FIG. 6**

FIG. 7

FIG. 8

**FIG. 9**

**FIG. 10A**

**FIG. 10B**

**FIG. 10C**

**FIG. 11**

**FIG. 12**

Collecting physiological signals using a group of electrodes fitting a body of a user ⟋ 1310

Reading the physiological signals using a processing circuit in a time-sharing mode, the processing circuit having different input impedances in different time periods of the time-sharing mode, the physiological signals read by the processing circuit including motion artifact signals of different proportions, and the processing circuit processing the physiological signals read by the processing circuit based on the motion artifact signals of different proportions ⟋ 1320

## FIG. 13

Collecting physiological signals using two groups of electrodes fitting a body of a user ⟋ 1410

Reading the physiological signals collected by the two groups of electrodes using a processing circuit, the processing circuit having different input impedances when reading the physiological signals collected by different groups of electrodes, the physiological signals collected by different groups of electrodes read by the processing circuit including motion artifact signals of different proportions, and the processing circuit processing the physiological signals based on the motion artifact signals of different proportions ⟋ 1420

## FIG. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3858232 A1

**Non-patent literature cited in the description**

- **AN XIANG et al.** *Adaptive Motion Artifact Reduction in Wearable ECG Measurements Using Impedance Pneumography Signal*

- **ZHANG HUANQIAN et al.** *A multi-channel electrode tissue impedance detection approach for motion artifact suppression in ambulatory electrocardiography*